# EUROPÄISCHE PATENTSCHRIFT

(11) **EP 3 849 642 B1**
(45) Veröffentlichungstag und Bekanntmachung des Hinweises auf die Patenterteilung: **26.10.2022**
(21) Anmeldenummer: 19769418.5
(22) Anmeldetag: 10.09.2019
(51) Int. Cl.: A61M 16/06

(54) **NASENBRILLE MIT VERBESSERTER, AUCH ASYMMETRISCHER STRÖMUNGSFÜHRUNG**
NASAL CANNULA HAVING IMPROVED AND ASYMMETRICAL FLOW CONTROL
LUNETTE NASALE À GUIDAGE DE FLUX AMÉLIORÉ ET ASYMÉTRIQUE

(30) Priorität: 14.09.2018 DE 102018122516
(43) Veröffentlichungstag der Anmeldung: 21.07.2021
(73) Patentinhaber: Hamilton Medical AG, 7402 Bonaduz (CH)
(72) Erfinder: SCHWAIGER, Ingo, 7000 Chur (CH); WALKER, Sandro, 7000 Chur (CH)
(74) Vertreter: Ruttensperger Lachnit Trossin Gomoll
(86) Internationale Anmeldenummer: PCT/EP2019/074124
(87) Internationale Veröffentlichungsnummer: WO 2020/053220

(56) Entgegenhaltungen:
- WO-A1-2004/073778
- WO-A1-2014/142681
- US-A1- 2014 158 127

## Beschreibung

Die vorliegende Anmeldung betrifft eine Nasenbrille zur nasalen Versorgung eines Patienten mit Therapiegas, umfassend ein Verteilkanalbauteil mit einem Verteilkanal, welcher längs einer virtuellen, ihn zentral durchsetzenden Verteilbahn verläuft, wobei die Verteilbahn eine Längsrichtung definiert, wobei der Verteilkanal zwei Abzweigöffnungen aufweist, wobei ausgehend von jeder Abzweigöffnung je ein Versorgungskanal zur Versorgung des Patienten mit Therapiegas vom Verteilkanal abzweigt, wobei der Verteilkanal zusätzlich zu den Abzweigöffnungen zwei mit Abstand von den Abzweigöffnungen angeordnete Kopplungsöffnungen aufweist, wobei die Nasenbrille einen Stopfen umfasst, durch welchen eine der beiden Kopplungsöffnungen als Verschlussöffnung durch den Stopfen für eine Gasdurchströmung verschließbar ist und wobei eine weitere der beiden Kopplungsöffnungen als Zufuhröffnung zum Anschluss einer Gas-Zufuhrleitung an den Verteilkanal ausgebildet ist.

Eine solche Nasenbrille ist aus der US 3 754 552 A bekannt. Der große Vorteil dieser bekannten Nasenbrille liegt darin, dass jede der beiden Kopplungsöffnungen Zufuhr- und Verschlussöffnung sein kann, je nachdem, in welche der beiden Kopplungsöffnungen der Stopfen eingeführt wird und je nachdem, an welche andere der beiden Kopplungsöffnungen eine Gas-Zufuhrleitung angeschlossen wird. Somit gestattet die bekannte Nasenbrille, einem Patienten von dessen rechter oder von dessen linker Köperseite her Therapiegas zuzuleiten, je nachdem, welche der beiden Seiten für die Zuleitung von Therapiegas abhängig von der jeweiligen Patientensituation vorteilhafter ist. Der Stopfen dient dazu, die nicht zur Gaszufuhr benötigte Kopplungsöffnung zu verschließen, um einen unerwünschten Austritt von Therapiegas aus dem Verteilkanal zu vermeiden, das dann nicht aus den Abzweigöffnungen ausströmen und somit den Patienten nicht erreichen würde.

Um die Kopplungsöffnungen am bekannten Verteilkanalbauteil zur Ankopplung des Stopfens und einer Gas-Zufuhrleitung ausreichend sicher ausbilden zu können, verläuft das Verteilkanalbauteil längs der Verteilbahn beiderseits der Abzweigöffnungen über eine verhältnismäßig große Strecke. Nachteilig an der bekannten Nasenbrille ist, dass im Bereich zwischen der durch den Stopfen als Verschlussöffnung genutzten Kopplungsöffnung und den Abzweigöffnungen ein verhältnismäßig großer Totraum gebildet ist, in welchem Therapiegas bei einer Versorgung eines Patienten unkontrolliert strömt und verwirbelt, was zu unvorteilhaft unvorhersagbaren Gasaustrittsverhältnissen an den Abzweigöffnungen führt.

Es ist daher Aufgabe der vorliegenden Anmeldung, die eingangs genannte Nasenbrille derart zu verbessern, dass trotz Verwendung eines Stopfens in einer als Verschlussöffnung genutzten Kopplungsöffnung sich die Strömungsverhältnisse im Verteilkanal definierter als bisher ausbilden und somit zum einen geringere Strömungsverluste im Verteilkanal auftreten und zum anderen ein definierteres Ausströmen von Therapiegas durch die Abzweigöffnungen zum Patienten hin erfolgt.

Diese Aufgabe löst die vorliegende Erfindung durch eine Nasenbrille der eingangs genannten Art, bei welcher der Stopfen eine Strömungsleitformation mit einer freiliegenden Strömungsleitfläche aufweist, wobei die Strömungsleitfläche in einem Bezugszustand, in dem der Stopfen bestimmungsgemäß die Verschlussöffnung verschließend an der Nasenbrille angeordnet ist, im Verteilkanal im Längserstreckungsbereich wenigstens einer Abzweigöffnung angeordnet ist und derart zur Verteilbahn angestellt ist, dass sie im Verteilkanal von der Zufuhröffnung her längs der Verteilbahn anströmendes Therapiegas zu wenigstens einer der Abzweigöffnungen hin umlenkt.

Sofern in der vorliegenden Anmeldung im Einzelfall nichts Abweichendes ausgesagt ist, ist die Nasenbrille der vorliegenden Anmeldung in dem oben bezeichneten Bezugszustand beschrieben.

Die freiliegende Strömungsleitfläche der Strömungsleitformation, welche im Bezugszustand dem von der Zufuhröffnung her anströmenden Therapiegas zugewandt und durch diese benetzbar ist, kann das von der Zufuhröffnung her anströmende Therapiegas möglichst verlustfrei zu der Abzweigöffnung hin umlenken, in deren Längserstreckungsbereich die Strömungsleitfläche angeordnet ist. Zur Erfüllung dieser Umlenkfunktion ist die Strömungsleitfläche relativ zur Verteilbahn angestellt, d. h. die Verteilbahn schließt mit der Strömungsleitfläche einen Winkel ein. Im einfachsten Fall kann die Strömungsleitfläche eine ebene oder wenigstens eine abschnittsweise ebene Fläche sein. Für eine besonders verlustfreie Umlenkung der Therapiegasströmung ist die Strömungsleitfläche jedoch bevorzugt eine wenigstens abschnittsweise gekrümmte Fläche. Die Strömungsleitfläche kann längs ihrer gesamten Längserstreckung längs der Verteilbahn gekrümmt ausgebildet sein.

In der Regel ist eine Umlenkung der zunächst längs der Verteilbahn verlaufenden Therapiegasströmung zur Abzweigöffnung um etwa 90°, also etwa im rechten Winkel, wünschenswert. Hierfür ist es zur möglichst verlustfreien Umlenkung vorteilhaft, wenn die Strömungsleitfläche eine konkave Krümmung aufweist. Für eine möglichst verlustfreie Umlenkung der Therapiegasströmung im Verteilkanalbauteil ist eine möglichst kontinuierlich gekrümmte Strömungsleitfläche von Vorteil, weshalb die Strömungsleitfläche bevorzugt über ihre gesamte Erstreckungslänge eine konkave Krümmung aufweisen kann. Um die Umlenkung um 90° ausgehend von der Verteilbahn zur Abzweigöffnung hin zu erreichen, ist die Strömungsleitfläche vorzugsweise um eine zur Verteilbahn orthogonale Krümmungsachse wenigstens abschnittsweise konkav gekrümmt.

Die Strömungsleitfläche kann, um an beiden Abzweigöffnungen möglichst einheitliche Therapiegas-Mengenströme bereitzustellen, unterschiedlich gekrümmte Abschnitte aufweisen. Da die Abzweigöffnungen in der Regel unterschiedliche Entfernung zu der Strömungsleitfläche aufweisen, ist die Erzielung betragsmäßig gleicher Mengenströme in beiden Abzweigöffnungen schwierig, aber gemäß einer bevorzugten Weiterbildung etwa dadurch erreichbar, dass die Strömungsleitfläche längs der Verteilbahn zwei konkav gekrümmte Abschnitte aufweist, welche zwischen sich einen konvex gekrümmten Abschnitt aufweisen. Bevorzugt weist die Strömungsleitfläche nur diese Abschnitte auf und besteht aus diesen. Eine derart mehrfach unterschiedlich gekrümmte Strömungsleitfläche ist bevorzugt in jedem Abschnitt um eine zur Verteilbahn orthogonale Krümmungsachse gekrümmt. In Breitenrichtung des Verteilkanals, also orthogonal zur Verteilbahn ist die Strömungsleitfläche bevorzugt ungekrümmt.

Auch dann, wenn die Strömungsleitfläche um die genannte zur Verteilbahn orthogonale Krümmungsachse gekrümmt ist, können sich in den wandnahen Bereichen des Verteilkanals Wirbel ausbilden, wenn die Strömungsleitfläche beispielsweise mit einem Winkel unter Bildung einer Ecke in die Wand des Verteilkanals mündet. Zur Vermeidung derartiger unerwünschter Wirbelbildungen ist gemäß einer bevorzugten Weiterbildung der vorliegenden Erfindung vorgesehen, dass die um eine zur Verteilbahn orthogonale Krümmungsachse konkav gekrümmte Strömungsleitfläche auch um eine längs der Verteilbahn verlaufende Krümmungsachse gekrümmt ist. Bevorzugt weist diese Strömungsleitfläche nur konkav gekrümmte Flächenbereiche, jedoch keine konvex gekrümmten Flächenbereiche auf.

Grundsätzlich kann das Therapiegas aus den Abzweigöffnungen unmittelbar nach Durchtritt durch diese im freien Strahl vom Verteilkanalbauteil zum Patienten, welcher die Nasenbrille trägt, hin ausströmen. Üblicherweise wird der Verteilkanal jedoch mit gewisser Entfernung von den Nasenöffnungen getragen. Zur gezielten Zuleitung von Therapiegas weist die Nasenbrille die Versorgungskanäle auf, welche ausgehend von den Abzweigöffnungen vom Verteilkanalbauteil abstehen. Diese Versorgungskanäle reichen in der Therapie an die Nasenöffnungen eines Patienten heran oder verlaufen sogar durch die Nasenöffnungen hindurch ein Stück weit in die Nasenhöhlen hinein. Von jeder Abzweigöffnung geht je ein Versorgungskanal aus. Die Versorgungskanäle können identisch bzw. zu einer die Verteilbahn zwischen den Abzweigöffnungen orthogonal schneidenden Symmetrieachse spiegelsymmetrisch ausgebildet sein.

Die Strömungsleitfläche verläuft wenigstens im Längserstreckungsbereich, also im Erstreckungsbereich längs der Verteilbahn, der der jeweiligen Verschlussöffnung näher gelegenen Abzweigöffnung. Dadurch kann sichergestellt werden, dass die Strömungsleitfläche eine Therapiegasströmung wenigstens zu dieser der Verschlussöffnung näher gelegenen Abzweigöffnung wirkungsvoll und gezielt umlenken kann. Es soll jedoch nicht ausgeschlossen sein, dass sich die Strömungsleitfläche auch in den Längserstreckungsbereich der der jeweiligen Verschlussöffnung ferner liegenden Abzweigöffnung hinein erstreckt oder über diese sogar vollständig hinweg verläuft. In diesem Falle wird die Umlenkung der von der Zufuhröffnung längs der Verteilbahn anströmenden Therapiegasströmung über einen langen Bereich des Verteilkanals schonend und mit nur geringen Verlusten erreicht.

Die wenigstens abschnittsweise konkave oder/und konvexe Krümmung der Strömungsleitfläche um eine jeweilige zur Verteilbahn orthogonale Krümmungsachse kann sich betragsmäßig längs ihres Verlaufs ändern. Bevorzugt nimmt die konkave Krümmung eines konkav gekrümmten Abschnitts der Strömungsleitfläche um eine zur Verteilbahn orthogonale Krümmungsachse mit zunehmender Annäherung an die zugeordnete Abzweigöffnung zu, d. h. der von der genannten Krümmungsachse ausgehende Krümmungsradius wird mit zunehmender Annäherung der Strömungsleitfläche an die der Strömungsleitfläche zugeordnete Abzweigöffnung kürzer.

Zur Einleitung von Therapiegas in den Verteilkanal des Verteilkanalbauteils weist die Nasenbrille bevorzugt einen Anschlussstutzen auf, welcher zum Anschluss einer Gas-Zufuhrleitung in die Zufuhröffnung einführbar ist. Zur Vermeidung von Wirbel bildenden Stufen im Verteilkanal bzw. längs einer die Therapiegasströmung im Verteilkanalbauteil führenden Wandung, kann gemäß einer bevorzugten Weiterbildung der vorliegenden Erfindung eine eine Gasströmung durch den Anschlussstutzen leitende Innenfläche des Anschlussstutzens bei bestimmungsgemäßer, betriebsbereiter Anordnung an der Nasenbrille mit der Strömungsleitfläche des Stopfens eine kontinuierliche strömungsleitende Fläche bilden. An der Stoßstelle der Innenfläche des Anschlussstutzens und der Strömungsleitfläche entsteht eine unvermeidliche Fuge, an der jedoch dann keine Wirbel entstehen, wenn die Innenfläche und die Strömungsleitfläche eine gemeinsame, über die Fuge hinweg bündige strömungsleitende Fläche bilden. Die gemeinsame kontinuierliche strömungsleitende Fläche ist bevorzugt an der genannten Fuge stufen- und knickfrei.

Zur Erleichterung der korrekten Anordnung von Stopfen und Anschlussstutzen relativ zueinander kann gemäß einer Weiterbildung der vorliegenden Erfindung vorgesehen sein, dass der Stopfen eine Formschlussformation aufweist und dass der Anschlussstutzen eine Formschlussgegenformation aufweist, wobei die Formschlussformation und die Formschlussgegenformation im betriebsbereiten Zustand miteinander in Formschlusseingriff stehen. Der Stopfen und der Anschlussstutzen können zwar an ihrer bezogen auf die Verteilbahn im betriebsbereiten Zustand radial äußere Außenseite eine konisch-kreiskegelförmige oder eine kreiszylindrische Außenwand, an ihrer radial inneren Innenfläche jedoch eine die Strömung nach radial außen begrenzende Wandung aufweisen, welche keinen Kreisquerschnitt aufweist. Dann, wenn die oben genannte kontinuierliche strömungsleitende Fläche gemeinsam durch die Innenfläche des Anschlussstutzens und die Strömungsleitfläche gebildet werden soll, kommt es besonders auf die richtige relative Anordnung von Stopfen und Anschlussstutzen zueinander an. Diese richtige relative Anordnung kann durch die genannten Formationen: Formschlussformation und Formschlussgegenformation, sicher erreicht werden. Um sicherzustellen, dass eine Fehlstellung von Anschlussstutzen und Stopfen relativ zueinander im betriebsbereiten Zustand ausgeschlossen ist, stehen die Formschlussformation und die Formschlussgegenformation vorzugsweise nur im betriebsbereiten Zustand miteinander in Formschlusseingriff.

Die Formschlussformation und die Formschlussgegenformation sind bevorzugt nach dem Schlüssel-Schloss-Prinzip ausgebildet. Hierzu kann eine der Formationen aus Formschlussformation und Formschlussgegenformation einen Vorsprung aufweisen und kann die jeweils andere Formation eine Ausnehmung aufweisen, in welche der Vorsprung nur dann eingeführt werden kann, wenn der Anschlussstutzen und der Stopfen relativ zueinander richtig orientiert sind, insbesondere hinsichtlich ihrer Winkelorientierung um die Verteilbahn relativ zueinander richtig orientiert sind.

Durch die Formschlussformation und die Formschlussgegenformation wird eine Verdrehsicherung von Anschlussstutzen und Stopfen relativ zueinander erreicht. Bei hergestelltem Formschlusseingriff ist eine Relativverdrehung von Stopfen und Anschlussstutzen relativ zueinander verhindert.

Da der Formschlusseingriff in der Regel im Verteilkanalbauteil hergestellt wird, und zwar an einem von einem Pflegepersonal bzw. vom behandelnden Arzt nicht einsehbaren Ort im Verteilkanalbauteil, kann eine oder können beide Formationen eine Einführhilfe zur Herstellung des Formschlusseingriffs aufweisen. So kann eine einen Vorsprung aufweisende Formation aus Formschlussformation und die Formschlussgegenformation zum frei auskragenden Ende hin verjüngt sein. Zusätzlich oder alternativ kann eine den Vorsprung aufnehmende Ausnehmung als die jeweils andere Formation aus Formschlussformation und Formschlussgegenformation vom Einführlängsende her in Einführrichtung des Vorsprungs verjüngend ausgebildet sein.

Nicht nur die relative Anordnung von Stopfen und Anschlussstutzen sind für die korrekte Funktion der hier diskutierten Nasenbrille vorteilhaft, sondern auch eine korrekte Lage des Anschlussstutzens relativ zum Verteilkanalbauteil. Eine korrekte Lage des Anschlussstutzens relativ zum Verteilkanalbauteil kann dadurch erreicht werden, dass der Anschlussstutzen eine Anordnungsformation aufweist und dass das Verteilkanalbauteil im Bereich der Zufuhröffnung eine Anordnungsgegenformation aufweist, wobei die Anordnungsformation und die Anordnungsgegenformation im betriebsbereiten Zustand miteinander in Formschlusseingriff stehen. Dieser Formschlusseingriff verhindert eine Verlagerung von Anschlussstutzen und Verteilkanalbauteil relativ zueinander aus der hergestellten korrekten Relativanordnung, insbesondere um die Verteilbahn herum.

Wiederum ist zum Ausschluss einer Fehlstellung von Anschlussstutzen und Verteilkanalbauteil bevorzugt, dass der beschriebene Formschlusseingriff zwischen Anordnungsformation und Anordnungsgegenformation nur im betriebsbereiten Zustand der Nasenbrille besteht bzw. nur bei einer zum betriebsbereiten Zustand führenden Montage der Nasenbrille herstellbar ist. Es gilt für die möglichen Ausgestaltungen der Anordnungsformation und der Anordnungsgegenformation das oben zur Formschlussformation und Formschlussgegenformation Gesagte entsprechend mit der Maßgabe, dass das für die Formschlussformation Gesagte für die Anordnungsformation gilt und dass das für die Formschlussgegenformation Gesagte für die Anordnungsgegenformation gilt. Daher kann eine Formation aus Anordnungsformation und Anordnungsgegenformation einen Vorsprung aufweisen und die jeweils andere Formation kann eine Ausnehmung aufweisen.

Da das Verteilkanalbauteil den Anschlussstutzen in der Regel radial außen umgibt oder/und verhältnismäßig dünnwandig ausgebildet ist, weist bevorzugt das Verteilkanalbauteil den Vorsprung und der Anschlussstutzen die Ausnehmung auf. Während eine Formation aus Formschlussformation und Formschlussgegenformation bevorzugt ein Vorsprung ist, welcher von der ihn tragenden Struktur parallel zur Verteilbahn auskragt, ist der Vorsprung einer Formation aus Anordnungsformation und Anordnungsgegenformation bevorzugt ein Radialvorsprung, welcher zur Verteilbahn hin vorsteht oder, ebenfalls möglich, von der Verteilbahn weg von einem ihn tragenden Bauteil vorsteht. Dementsprechend ist auch die Ausnehmung der jeweils anderen Formation eine Radialausnehmung. Der Vorsprung kann als Radialvorsprung als Einführhilfe längs der Verteilbahn mit abnehmender radialer Höhe oder/und mit abnehmender in Umfangsrichtung um die Verteilbahn zu messender Vorsprungsbreite ausgebildet sein.

Zur korrekten Anordnung auch des Stopfens relativ zum Verteilkanalbauteil kann der Stopfen eine Orientierungsformation aufweisen. Ebenso kann das Verteilkanalbauteil im Bereich der Verschlussöffnung eine Orientierungsgegenformation aufweisen, wobei die Orientierungsformation und die Orientierungsgegenformation im betriebsbereiten Zustand, aus den bereits genannten Gründen bevorzugt nur im betriebsbereiten Zustand, miteinander in Formschlusseingriff stehen.

Da sowohl der Stopfen als auch der Anschlussstutzen in der Regel axial, also längs der Verteilbahn in das Verteilkanalbauteil eingeführt oder im Falle des Anschlussstutzens auch auf das Verteilkanalbauteil aufgesteckt werden, gilt wegen der gemeinsamen Anordnungsbewegung von Stopfen und Anschlussstutzen relativ zum Verteilkanalbauteil das für die Anordnungsformation und Anordnungsgegenformation Gesagte ebenso für die Orientierungsformation und die Orientierungsgegenformation mit der Maßgabe, dass das für die Anordnungsformation Gesagte für die Orientierungsformation zutrifft und das für die Anordnungsgegenformation Gesagte für die Orientierungsgegenformation zutrifft. Dies schließt ein, dass auch das für die Formschlussformation Gesagte für die Orientierungsformation zutrifft und das für die Formschlussgegenformation Gesagte für die Orientierungsgegenformation zutrifft. Eine von beiden Formationen kann ein Vorsprung und die jeweils andere Formation kann eine den Vorsprung im Formschlusseingriff aufnehmende Ausnehmung sein. Dadurch kann eine Verdrehsicherung des Stopfens relativ zum Verteilkanalbauteil ebenso hergestellt werden wie eine Verdrehsicherung des Anschlussstutzens gegen eine Verdrehung relativ zum Verteilkanalbauteil um die Verteilbahn.

Um einen die Nasenbrille benutzenden Patienten je nach Behandlungssituation wahlweise von jeder seiner beiden Körperseiten her mit Therapiegas versorgen zu können, sind die beiden Kopplungsöffnungen gemäß einer Weiterbildung der vorliegenden Erfindung wenigstens insoweit gleichartig ausgebildet, dass jede der beiden Kopplungsöffnungen sowohl Verschlussöffnung als auch Zufuhröffnung sein kann. Wenigstens die die Kopplungsöffnungen aufweisenden Längsendbereiche des Verteilkanals können spiegelsymmetrisch ausgebildet sein.

Damit eine Anordnung von Stopfen oder/und Anschlussstutzen in egal welcher der Kopplungsöffnungen nicht zu unterschiedlichen Strömungsverhältnissen im Verteilkanal führt, sind der Stopfen oder/und der Anschlussstutzen bezüglich einer ihre jeweilige Längsachse enthaltenden Symmetrieebene bevorzugt spiegelsymmetrisch ausgebildet. Im Bezugszustand liegt die Verteilbahn wenigstens im Anordnungsbereich der Strömungsleitfläche in der Symmetrieebene des Stopfens. Entsprechend gilt für den Anschlussstutzen, dass wenigstens am Gasauslassende des Anschlussstutzens die Verteilbahn in der Symmetrieebene gelegen ist.

In manchen Therapiefällen ist eine asymmetrische Zufuhr von Therapiegas zum Patienten gewünscht. Da Therapiegas in das Verteilkanalbauteil über die Gas-Zufuhr nur an einer Stelle, der Zufuhröffnung, eingeleitet wird, muss eine asymmetrische Verteilung von Therapiegas innerhalb des Verteilkanalbauteils oder/und in den Versorgungskanälen, sofern vorhanden, bewirkt werden.

Es sind im Stand der Technik bereits Maßnahmen bekannt, um Therapiegas ausgehend von einer Nasenbrille asymmetrisch, d. h. in ungleichen Mengen pro Zeiteinheit an die einzelnen Nasenöffnungen eines Patienten abzugeben. Beispielsweise ist bekannt, Versorgungskanäle mit unterschiedlicher Länge oder/und unterschiedlichem Strömungsquerschnitt zu verwenden. Diese an den Versorgungskanälen verwirklichten Maßnahmen zur mengenmäßig ungleichmäßigen Verteilung von Therapiegas an die beiden Nasenöffnungen eines Patienten haben jedoch den Nachteil, dass sie in physiologischer Rückwirkung mit dem Patienten stehen. Der Patient wird möglicherweise den längeren von zwei ungleich langen Versorgungskanälen oder/und den Versorgungskanal mit größerem Öffnungsquerschnitt in seinen Nasenhöhlen spüren. Dies kann während der Therapie unangenehm werden und den Therapieerfolg gefährden.

Die vorliegende Erfindung stellt demgegenüber die Möglichkeit bereit, einem Patienten Therapiegas in ungleichen Mengen pro Zeiteinheit zuzuführen, bzw. durch die Abzweigöffnungen strömen zu lassen, ohne dass hierfür ungleich oder asymmetrisch gestaltete Versorgungskanäle notwendig wären. Es kann daher eine Nasenbrille mit bevorzugt spiegelsymmetrisch angeordneten Versorgungskanälen auch zur Verabreichung ungleich großer Therapiegas-Mengenströme verwendet werden. Die Versorgungskanal-Symmetrieebene der symmetrisch ausgebildeten Versorgungskanäle ist eine Ebene, welche auf halben Abstand zwischen den Abzweigöffnungen die Verteilkanalbahn orthogonal schneidet.

Eine solche Verabreichung von unterschiedlichen Therapiegas-Mengenströmen durch die beiden Abzweigöffnungen kann gemäß einer besonders bevorzugten Weiterbildung der vorliegenden Erfindung dadurch erreicht werden, dass der Stopfen im betriebsbereit in der Verschlussöffnung angeordneten Zustand die der Verschlussöffnung näher gelegene Abzweigöffnung wenigstens teilweise verschließt.

Bevorzugt sind die Abzweigöffnungen gleich groß bzw. bezüglich der oben genannten Versorgungskanal-Spiegelsymmetrieebene spiegelsymmetrisch, jedoch mit bevorzugt gleichem Öffnungsquerschnitt ausgebildet, so dass nicht nur grundsätzlich eine Verabreichung von betragsmäßig gleich großen Therapiegas-Mengenströmen durch die beiden Abzweigöffnungen möglich ist, sondern so dass auch eine Vertauschung von Verschlussöffnung und Zuführöffnung zu betragsmäßig gleichen Therapiegas-Mengenströmen durch die Abzweigöffnungen führt, jedoch an der jeweils anderen Abzweigöffnung als vor der Vertauschung.

Diese Ausbildung einer Nasenbrille mit einer inneren Strömungsleitfläche in einem Verteilkanal, welche von der Zuführöffnung her anströmendes Therapiegas zu einer Abzweigöffnung umlenkt und dabei gewünscht mengenmäßig unterschiedliche Therapiegas-Mengenströme durch die beiden Abzweigöffnungen bewirkt, ist derart bevorzugt, dass die Anmelderin sich vorbehält, Patentschutz anzustreben für eine Nasenbrille zur nasalen Versorgung eines Patienten mit Therapiegas, umfassend ein Verteilkanalbauteil mit einem Verteilkanal, welcher längs einer virtuellen, ihn zentral durchsetzenden Verteilbahn verläuft, wobei die Verteilbahn eine Längsrichtung definiert, wobei der Verteilkanal zwei Abzweigöffnungen aufweist, wobei ausgehend von jeder Abzweigöffnung je ein Versorgungskanal zur Versorgung des Patienten mit Therapiegas vom Verteilkanal abzweigt, wobei der Verteilkanal zusätzlich zu den Abzweigöffnungen eine mit Abstand von den Abzweigöffnungen angeordnete Zufuhröffnung aufweist, welche zum Anschluss einer Gas-Zufuhrleitung an den Verteilkanal ausgebildet ist, wobei die Nasenbrille eine Strömungsleitformation mit einer im Verteilkanal freiliegenden Strömungsleitfläche aufweist, wobei die Strömungsleitfläche im Verteilkanal im Längserstreckungsbereich wenigstens einer Abzweigöffnung angeordnet ist und derart zur Verteilbahn angestellt ist, dass sie im Verteilkanal von der Zufuhröffnung her längs der Verteilbahn anströmendes Therapiegas zu wenigstens einer der Abzweigöffnungen hin umlenkt, wobei diejenige Abzweigöffnung, zu welcher die Strömungsleitfläche längs der Verteilbahn hin verläuft, eine kleinere Öffnungsquerschnittsfläche aufweist als die jeweils andere Abzweigöffnung.

Eine solche Nasenbrille kann zwei Nasenbrillenhälften aufweisen, welche relativ zueinander beweglich hergestellt werden und zu der Nasenbrille montiert werden können. Diese Nasenbrille, die im Gegensatz zur eingangs Genannten keinen Stopfen aufweist und bei der die Strömungsleitfläche unverlagerbar als Begrenzungswand des Verteilkanals vorgesehen ist, erzielt den gleichen Effekt wie die Nasenbrille mit teilweise durch den Stopfen verschlossener Abzweigöffnung: Eine der beiden Abzweigöffnungen weist eine kleinere Öffnungsquerschnittsfläche auf, so dass durch die Abzweigöffnung mit kleinerer Öffnungsquerschnittsfläche, das ist im Falle der stopfenbehafteten Nasenbrille die teilweise verschlossene Abzweigöffnung, pro Zeiteinheit eine kleinere Menge an Therapiegas austritt als durch die andere Abzweigöffnung mit größerer Öffnungsquerschnittsfläche.

Zur Unterstützung einer Umlenkung von von der Zufuhröffnung her anströmendem Therapiegas zu den beiden Abzweigöffnungen hin kann in Weiterbildung der vorliegenden Erfindung die oben genannte Strömungsleitformation eine erste Strömungsleitformation sein, welche von der Zufuhröffnung her längs der Verteilbahn anströmendes Therapiegas zu einer der Abzweigöffnungen hin umlenkt, wobei der Stopfen eine zweite Strömungsleitformation mit einer zweiten Strömungsleitfläche aufweist, welche längs der Verteilbahn mit Abstand von der ersten Strömungsleitformation angeordnet ist und im Bezugszustand von der Zufuhröffnung her längs der Verteilbahn anströmendes Therapiegas zu der jeweils anderen Abzweigöffnung hin umlenkt.

Die erste und die zweite Strömungsleitfläche bilden bevorzugt keine zusammenhängende Leitfläche, so dass sie bevorzugt nicht Abschnitte ein und derselben Leitfläche sind.

Das oben zur konstruktiven Ausgestaltung der ersten Strömungsleitformation mit der ersten Strömungsleitfläche Gesagte gilt auch für die zweite Strömungsleitformation mit der zweiten Strömungsleitfläche. Insbesondere kann die zweite Strömungsleitfläche, ebenso wie die erste Strömungsleitfläche, um wenigstens eine der genannten Krümmungsachsen konkav gekrümmt sein, um längs der Verteilbahn das anströmende Therapiegas an deren Abzweigöffnung hin umzulenken. Bevorzugt ist die zweite Strömungsleitfläche betragsmäßig kleiner als die erste Strömungsleitfläche und bevorzugt ist die zweite Strömungsleitfläche nur um eine oder um zwei zueinander orthogonale Krümmungsachsen konkav gekrümmt. Dies vermeidet eine unerwünschte Geräuschbildung im Verteilkanal während eines Therapiebetriebs.

Die zweite Strömungsleitformation, welche bezüglich einer von der Zuführöffnung her längs der Verteilbahn anströmenden Therapiegasströmung stromaufwärts der ersten Strömungsleitformation angeordnet ist, stellt für die Strömung von Therapiegas zur ersten Strömungsleitformation ein Strömungshindernis dar. Daher kann in konkreterer konstruktiver Ausgestaltung die zweite Strömungsleitformation sich nur über einen Teilquerschnitt des Verteilkanals erstrecken und in ihrem Erstreckungsbereich eine längs der Verteilbahn anströmende Gasströmung in die jeweils andere Abzweigöffnung umlenken und im Bezugszustand außerhalb ihres Erstreckungsbereichs mit dem Verteilkanal eine Durchströmöffnung für eine Strömung von Therapiegas an der zweiten Strömungsleitformation vorbei zur ersten Strömungsleitfläche bilden. Durch diese Durchströmöffnung hindurch kann ein Anteil des von der Zufuhröffnung her anströmenden Therapiegases zur ersten Strömungsleitformation strömen.

Um eine möglichst effektive Umlenkung eines Therapiegasströmungsanteils durch die zweite Strömungsleitfläche in die jeweils andere Abzweigöffnung zu bewirken, ist es vorteilhaft, wenn die zweite Strömungsleitformation über einen vorbestimmten Winkelbereich um die Verteilbahn hinweg an einer Innenwandung des Verteilkanals anliegt. Dann existiert zumindest in dem Winkelbereich kein Spalt zwischen der zweiten Strömungsleitformation, insbesondere der zweiten Strömungsleitfläche, und der Innenwandung des Verteilkanals. Ein solcher Spalt ist unerwünscht, da durch ihn hindurch Therapiegas strömen kann, anstatt von der zweiten Strömungsleitfläche in die jeweils andere Abzweigöffnung umgelenkt zu werden. Der Winkelbereich kann aus mehreren mit Abstand voneinander gelegenen Teilwinkelbereichen gebildet sein, etwa wenn sich der Winkelbereich über die jeweils andere Abzweigöffnung hinweg erstreckt, wo sich wegen der jeweils anderen Abzweigöffnung keine Innenwandung des Verteilkanals befindet. Somit kann auch die zweite Strömungsleitformation die ihr zugeordnete jeweils andere Abzweigöffnung wenigstens teilweise verdecken, um bei vorgegebener Therapiegasströmung im Verteilkanal den durch die jeweils andere Abzweigöffnung strömenden Therapiegas-Mengenstrom mengenmäßig zu beeinflussen.

Sind betragsmäßig verschiedene Therapiegas-Mengenströme durch die beiden Abzweigöffnungen oder/und aus den Versorgungskanälen heraus gewünscht, kann dies zusätzlich oder alternativ zu der oben bereits genannten Maßnahme einer teilweisen Verdeckung einer Abzweigöffnung auch dadurch erreicht werden, dass eine von Therapiegas durchströmbare Querschnittsfläche der Durchströmöffnung größer oder kleiner als die Hälfte Querschnittsfläche des Verteilkanals am Ort der Durchströmöffnung ist.

Bevorzugt ist die Durchstromöffnung entweder größer als 70% der Querschnittsfläche des Verteilkanals am Ort der Durchströmungsöffnung oder kleiner als 30% der Querschnittsfläche des Verteilkanals, um deutlich mengenmäßig voneinander verschiedene Therapiegas-Mengenströme durch die beiden Abzweigöffnungen hindurch zu erzeugen.

Die Ausbildung einer zweiten Strömungsleitformation wie sie oben genannt ist, kann auch an der oben genannten stopfenfreien Nasenbrille realisiert sein, etwa wenn diese aus zwei Nasenbrillenbauteilen im Spritzgussverfahren gefertigt wird.

Die eingangs genannte stopfenbehaftete Nasenbrille kann dann besonders einfach in einen betriebsbereiten Zustand versetzt werden, wenn das Verteilkanalbauteil aus einem Material gebildet ist, welches einen niedrigeren Elastizitätsmodul aufweist als das Material des Stopfens oder/und des Anschlussstutzens, sodass das Verteilkanalbauteil beim Einführen eines Einführbauteils aus Stopfen oder/und Anschlussstutzens in das Verteilkanalbauteil durch das jeweilige Einführbauteil verformbar ist.

Zur möglichst einfachen Anschließbarkeit des Anschlussstutzens an die dann als Zufuhröffnung genutzte Kopplungsöffnung befinden sich gemäß einer bevorzugten Weiterbildung der vorliegenden Erfindung die beiden Kopplungsöffnungen an entgegengesetzten Längsenden des Verteilkanals. Bevorzugt verläuft die Verteilbahn zwischen den beiden Kopplungsöffnungen geradlinig, um unerwünschte Umlenkverluste und Geräuschbildung zu vermeiden. Zusätzlich oder alternativ sind die beiden Abzweigöffnungen in Längsrichtung mittig zwischen den Kopplungsöffnungen gelegen, damit unabhängig davon, welche Kopplungsöffnung als Zufuhröffnung gewählt ist, bei sonst gleichen Betriebsbedingungen sich im Verteilkanal die gleichen, jedoch seitenverkehrten Strömungsverhältnisse ausbilden.

Die vorliegende Erfindung betrifft außerdem einen Nasenbrillen-Therapiebausatz, umfassend eine Nasenbrille, wie sie oben beschrieben ist, und wenigstens zwei oder bevorzugt mehr als zwei Stopfen, welche sich in der Länge der ersten oder/und der zweiten Strömungsleitformation oder/und in der Gestalt der ersten oder/und der zweiten Strömungsleitfläche oder/und in der Größe oder/und Gestalt einer von der zweiten Strömungsleitformation und dem Verteilkanal gebildeten Durchströmöffnung für eine Strömung von Therapiegas an der zweiten Strömungsleitformation vorbei unterscheiden.

Mit dem Nasenbrillen-Therapiebausatz kann mit einem einzigen Verteilkanalbauteil und wenigstens zwei Stopfen, vorzugsweise einer Mehrzahl von Stopfen, die Nasenbrille durch Auswahl eines jeweiligen verwendeten Stopfens aus der Mehrzahl von verfügbaren Stopfen hinsichtlich ihrer Strömungsbedingungen an den jeweiligen Therapiefall individuell angepasst werden. Dadurch, dass sich die Stopfen in der genannten Weise unterscheiden, beispielsweise durch unterschiedliche Länge von Stopfen, kann das Ausmaß, in welchem eine Abzweigöffnung durch den Stopfen bzw. durch eine Strömungsleitformation verdeckt wird, verändert werden. Zusätzlich oder alternativ kann durch Auswahl eines Stopfens hinsichtlich einer Gestalt einer Strömungsleitfläche die Umlenkung des anströmenden Therapiegases zu der der jeweiligen Strömungsleitfläche zugeordneten Abzweigöffnung verändert werden.

Weiter kann zusätzlich oder alternativ durch Auswahl eines Stopfens aus einer Mehrzahl von Stopfen mit unterschiedlichen Durchströmöffnungen der Strömungsanteil an Therapiegas, welches den beiden Abzweigöffnungen zugeleitet wird, verändert werden. Somit kann eine Nasenbrille durch Auswahl eines Stopfens aus einer Mehrzahl von Stopfen in sehr einfacher Weise auf sehr viele unterschiedliche Therapieanwendungen hin angepasst werden.

Die vorliegende Erfindung wird nachfolgend anhand der beiliegenden Zeichnungen näher erläutert werden. Es stellt dar:
- Fig. 1: eine schematische Explosionsansicht einer ersten Ausführungsform einer erfindungsgemäßen Nasenbrille,
- Fig. 2: eine schematische Längsschnittansicht durch die Ausführungsform von Fig. 1, wobei die Schnittebene die Verteilbahn enthält,
- Fig. 3: eine der Ansicht von Fig. 2 entsprechende Längsschnittansicht einer zweiten Ausführungsform einer erfindungsgemäßen Nasenbrille mit einen von der Nasenbrille von Fig. 2 abweichenden Stopfen,
- Fig. 4: eine den Ansichten der Fig. 2 und 3 entsprechende weitere Längsschnittansicht durch eine dritte erfindungsgemäße Ausführungsform einer Nasenbrille mit einem noch weiteren verschiedenen Stopfen und
- Fig. 5: eine teilgeschnittene perspektivische Ansicht des Verteilbauteils mit einer Unteransicht des Stopfens der ersten Ausführungsform.

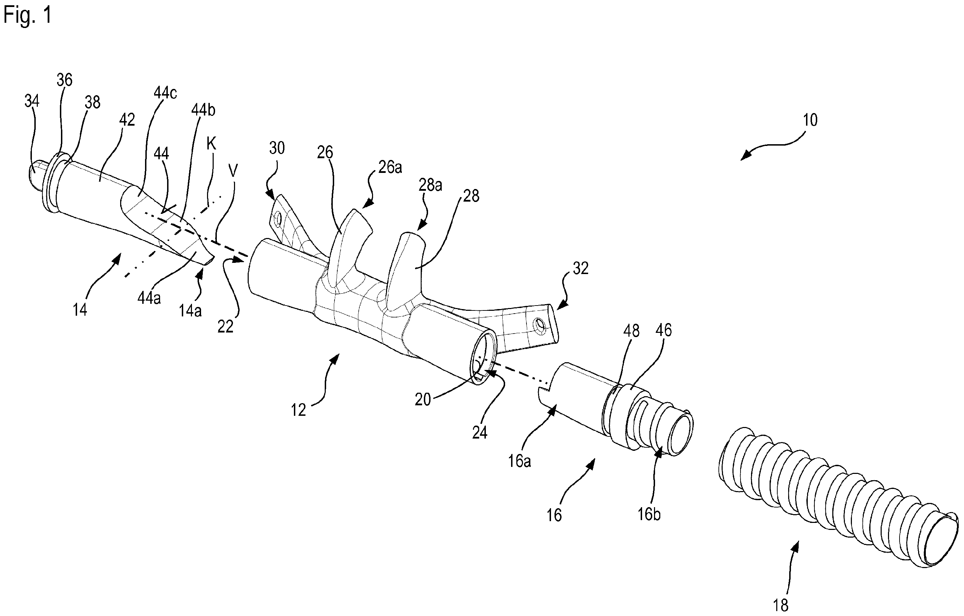

In Fig. 1 ist eine erste Ausführungsform einer erfindungsgemäßen Nasenbrille allgemein mit 10 bezeichnet. Die Nasenbrille 10 umfasst ein Verteilkanalbauteil 12, einen Stopfen 14 und einen Anschlussstutzen 16. An das vom Verteilkanalbauteil 12 betriebsmäßig abgewandte Längsende 16b des Anschlussstutzens 16 ist ein Zufuhrschlauch 18 anbringbar, insbesondere aufschraubbar.

Das Verteilkanalbauteil 12 weist einen sich längs einer Verteilbahn V erstreckenden Verteilkanal 20 auf. An bezüglich der Verteilbahn V entgegengesetzten axialen Längsenden des Verteilkanalbauteils 12 ist je eine Kopplungsöffnung 22 bzw. 24 ausgebildet, durch welche hindurch der Verteilkanal 20 zugänglich ist. Vom Verteilkanalbauteil 12 zweigen zwei Versorgungskanäle 26 und 28 ab, welche in den Verteilkanal 20 des Verteilkanalbauteils 12 münden. Mittels Trägerstegen 30 und 32 kann das Verteilkanalbauteil 12 und damit die Nasenbrille 10 von einem Patienten in an sich bekannter Weise angelegt und getragen werden.

Das Verteilkanalbauteil 12 einschließlich der Versorgungskanäle 26 und 28 sowie der Trägerstege 30 und 32 ist bevorzugt einstückig ausgebildet, etwa durch Spritzgießen.

Die Versorgungskanäle 26 und 28 sind im Therapiebetrieb der Nasenbrille 10 mit ihren vom Verteilkanal 20 fernen Längsenden 26a bzw. 28a nahe an Nasenöffnungen eines Patienten herangeführt oder treten durch die Nasenöffnungen in die Nasenhöhlen des Patienten ein.

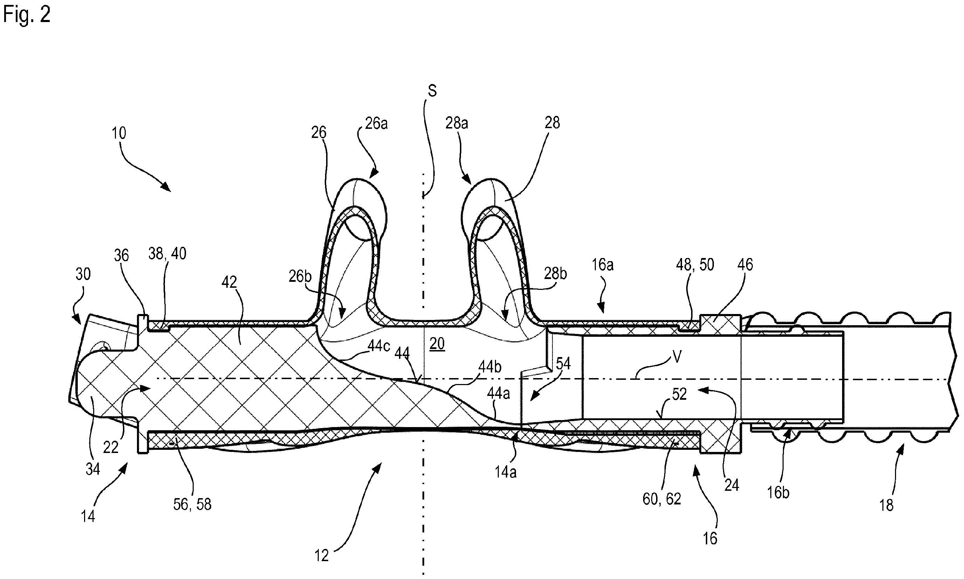

Das Verteilkanalbauteil 12 ist bezüglich einer die geradlinige Verteilbahn V orthogonal in der Abstandsmitte zwischen den Versorgungskanälen 26 und 28 schneidenden Symmetrieebene S spiegelsymmetrisch ausgebildet (s. Fig. 2). Diese spiegelsymmetrische Ausbildung hat den Vorteil, dass die Kopplungsöffnungen 22 und 24 identisch ausgebildet sind und somit jede sowohl vom Stopfen 14 als auch vom Anschlussstutzen 16 nutzbar ist.

In der in Fig. 1 gezeigten Explosionsansicht ist die Nasenbrille 10 zur Gaszufuhr von der linken Seite eines die Nasenbrille 10 tragenden Patienten konfiguriert. Die Kopplungsöffnung 24 ist daher eine Zufuhröffnung 24.

Um einen unerwünschten Austritt von Therapiegas aus dem Verteilkanal 20 durch die Kopplungsöffnung 22 zu vermeiden, ist der Stopfen 14 zur Anordnung im Verteilkanal 20 durch die Kopplungsöffnung 22 hindurch angeordnet und vorgesehen. Die Kopplungsöffnung 22 bildet daher eine Verschlussöffnung 22.

Der bevorzugt einstückig ausgebildete, durch Spritzgießen hergestellte Stopfen 14 weist an seinem einen Längsende einen Griffansatz 34 auf, welcher von einer Stoppscheibe 36 absteht, die eine größere Fläche aufweist als die lichte Querschnittsfläche der Kopplungsöffnung 22. Die Stoppscheibe 36 überragt somit einen Rand der Kopplungsöffnung 22 und verhindert so körperlich ein übermäßiges Einführen des Stopfens 14 in den Verteilkanal 20. Die Stoppscheibe 36 liegt im betriebsbereiten Zustand der Nasenbrille 10 an einem die Kopplungsöffnung 22 begrenzenden Rand des Verteilkanalbauteils 12 an und bildet so einen mechanischen Anschlag mit dem Verteilkanalbauteil 12.

Auf der dem Griffansatz 34 entgegengesetzten Seite der Stoppscheibe 36 ist eine Rastnut 38 ausgebildet, in welche im betriebsbereiten Zustand der Nasenbrille 10 ein um die Verteilbahn V umlaufender Radialvorsprung 40 des Verteilkanalbauteils 12 verrastend eingreift (siehe Fig. 2). Der Radialvorsprung 40 und die Rastnut 38 bilden einen überwindbaren Formschluss-Rasteingriff, welcher ein unerwünschtes Lösen des Stopfens 14 vom Verteilkanalbauteil 12, etwa durch den im Verteilkanal 20 herrschenden Therapiegasstrom, verhindert.

An die Rastnut 38 schließt in Richtung zum Einführlängsende 14a des Stopfens 14 eine Strömungsleitformation 42 an. Auf ihrer vom Griffansatz 34 weg weisenden Seite ist die Strömungsleitformation 42 durch eine Strömungsleitfläche 44 begrenzt, welche dann, wenn der Stopfen 14 in das Verteilkanalbauteil 12 längs der Verteilbahn V durch die Kopplungsöffnung 22 hindurch eingeführt ist, eine den Strömungsraum des Verteilkanals 20 begrenzende Wand bildet. Die Strömungsleitfläche 44 wird im Therapiebetrieb von Therapiegas angeströmt, das aus dem Anschlussstutzen 16 zur Versorgung des Patienten mit Therapiegas austritt.

Die Strömungsleitfläche 44 ist im vorliegenden ersten Ausführungsbeispiel einachsig gekrümmt, und zwar ausschließlich um Krümmungsachsen, welche orthogonal zur Verteilbahn V angeordnet sind. Eine beispielhafte Krümmungsachse K ist in Fig. 1 angezeigt. Längs der Krümmungsachse K ist die Strömungsleitfläche 44 im dargestellten Ausführungsbeispiel ungekrümmt, d. h. sie weist geradlinige Randlinien in Schnittebenen orthogonal zur Verteilbahn V auf.

Die Strömungsleitfläche 44 weist in dem in Fig. 1 dargestellten ersten Ausführungsbeispiel drei Bereiche mit unterschiedlicher Krümmungsart auf, nämlich einen dem Einführlängsende 14a nächstgelegenen Bereich 44a mit konkaver Krümmung, einen sich längs der Verteilbahn V daran anschließenden Bereich 44b mit konvexer Krümmung und einen sich wiederum an diesen anschließenden dritten Bereich 44c mit einer konkaven Krümmung. Somit weist die Strömungsleitfläche 44 zwei konkave Krümmungsbereiche 44a und 44c und einen längs der Verteilbahn V dazwischen liegenden konvexen Krümmungsabschnitt 44b auf. Mit zunehmender Annäherung des konkaven Krümmungsbereichs 44c an den Versorgungskanal 26 (oder auch 28, je nachdem, durch welche Kopplungsöffnung 22 oder 24 der Stopfen 14 in den Verteilkanal 20 eingeführt wird) nimmt die Krümmung des dritten Krümmungsbereichs 44c zu, d. h. dessen Krümmungsradius wird kleiner. Dies dient der verlust- und geräuscharmen Umleitung einer zunächst axialen, längs der Verteilbahn V strömenden Therapiegasströmung in den der Strömungsleitfläche 44 des Stopfens 14 zugeordneten Versorgungskanal 26 in dem in Fig. 1 dargestellten Beispiel.

Der Stopfen 14 ist, damit er durch jede der beiden Kopplungsöffnungen 22 und 24 hindurch mit gleicher strömungsleitenden Wirkung im Verteilkanal 20 anordenbar ist, bezüglich einer Symmetrieebene symmetrisch, welche parallel zur Verteilbahn V und orthogonal zur Krümmungsachse K orientiert ist.

Der Anschlussstutzen 16 ist mit einem Einführabschnitt 16a im in Fig. 1 gezeigten Beispiel durch die Kopplungsöffnung 24 hindurch in den Verteilkanal 20 des Verteilkanalbauteils 12 einführbar. An den entgegengesetzten Anbindungsabschnitt 16b ist der Zufuhrschlauch 18 anbindbar, insbesondere aufschraubbar.

Zwischen den Abschnitten 16a und 16b weist der Anschlussstutzen 16 einen Stoppring 46 auf, welcher einen die Kopplungsöffnung 24 begrenzenden Rand des Verteilkanalbauteils 12 radial überragt und so eine Einführtiefe des Anschlussstutzens 16 in den Verteilkanal 20 begrenzt. Zwischen dem Einführabschnitt 16a und dem Stoppring 46 weist auch der Anschlussstutzen 16 eine Rastnut 48 auf, in welche ein um die Verteilbahn V umlaufender Rastvorsprung 50 an dem der Kopplungsöffnung 24 näher gelegenen Endbereich des Verteilkanalbauteils 12 zur Bildung eines überwindbaren Formschluss-Rasteingriffs im betriebsbereiten Zustand der Nasenbrille 10 eingreift.

In Fig. 2 ist die Nasenbrille 10 von Fig. 1 in einer Längsschnittansicht längs einer die Verteilbahn V enthaltenden Schnittebene dargestellt. In Fig. 2 ist die Spiegelsymmetrieebene S des Verteilkanalbauteils 12 gezeigt. Diese ist orthogonal zur Verteilbahn V und zur Zeichenebene der Fig. 2.

Die Schnittebene des Längsschnitts durch die Nasenbrille 10 durchsetzt die Abzweigöffnungen 26b bzw. 28b, von welchen ausgehend sich die Versorgungskanäle 26 und 28 vom Verteilkanal 12 weg erstrecken. Die Abzweigöffnungen 26b und 28b weisen aufgrund der genannten Symmetriebedingungen die gleichen Querschnittsflächen auf, so dass grundsätzlich eine symmetrische Versorgung beider Nasenöffnungen eines Patienten durch die Versorgungskanäle 26 und 28 mit gleichen Therapiegas-Mengenströmen möglich ist. Eine Innenfläche 52 des Anschlussstutzens 16 bildet eine kontinuierliche strömungsleitende Fläche 54 mit der Strömungsleitfläche 44 des Stopfens 14. Die Innenfläche 52 und die Strömungsleitfläche 44 gehen bündig ineinander über.

Wie außerdem in Fig. 2 zu erkennen ist, ist die Strömungsleitfläche 44 des Stopfens 14 in Richtung orthogonal zur Zeichenebene der Fig. 2 ungekrümmt, was oben im Zusammenhang mit Fig. 1 bereits erwähnt wurde.

Der konkave Abschnitt 44c endet bei korrekt sitzendem Stopfen 14 am Rand der dem Stopfen 14 nächstgelegenen und daher zugeordneten Abzweigöffnung 26d. Die Strömungsleitfläche 44 und von dieser insbesondere der konkave Abschnitt 44c befinden sich im Längserstreckungsbereich der Abzweigöffnung 26b.

Die Strömungsleitfläche 44 erstreckt sich sogar längs der Verteilbahn V in den Längserstreckungsbereich der der Abzweigöffnung 28b. Somit befindet sich der konkave Abschnitt 44b der Strömungsleitfläche 44 größtenteils oder sogar zur Gänze in Längsrichtung längs der Verteilbahn V zwischen den Abzweigöffnungen 26b und 28b.

Wie aus Fig. 2 hervorgeht, könnten problemlos der Stopfen 14 und der Anschlussstutzen 16 ihre Positionen am Verteilkanalbauteil 12 tauschen, was zu einer spiegelbildlichen Versorgung mit Therapiegas durch die Längsenden 26a und 28a der Versorgungskanäle 26 und 28 führen würde.

Die in Fig. 2 gezeigte Konfiguration führt bei Anströmung mit Therapiegas von der Zufuhröffnung 24 her zu einer weitestgehend symmetrischen Durchströmung der Versorgungskanäle 26 und 28 mit Therapiegas, also zu einer Durchströmung beider Versorgungskanäle 26 und 28 mit etwa gleichen Therapiegas-Mengenströmen.

Der Radialvorsprung 40 an der Kopplungsöffnung 22 ist in Fig. 2 nicht vollständig umlaufend dargestellt, da an der Unterseite der den Verteilkanal 20 begrenzenden Wand des Verteilkanalbauteils 12 ein sich längs der Verteilbahn V erstreckender und nach radial innen zur Verteilbahn V hin vorspringender Radialvorsprung 56 als Orientierungsgegenformation ausgebildet ist. Dieser Radialvorsprung 56 greift in eine komplementäre Ausnehmung 58 als eine Orientierungsformation im Stopfen 14 ein. Sowohl die Orientierungsformation 58 in Gestalt der Ausnehmung als auch die Orientierungsgegenformation 56 in Gestalt des Radialvorsprungs verjüngen sich in der dargestellten Anordnungssituation in radialer Richtung zur Verteilbahn V hin und in axialer Richtung, von der Kopplungsöffnung 22 ausgehend, zur Längsmitte des Verteilkanals 20 hin. Dies erleichtert ein Einführen des Stopfens 14 in das Verteilkanalbauteil von der Kopplungsöffnung 22 her (oder, wegen der spiegelsymmetrischen Ausbildung des Verteilkanalbauteils 12 oder wenigstens des Verteilkanals 20, auch von der Kopplungsöffnung 24 her).

Der Formschlusseingriff zwischen Orientierungsformation 58 und Orientierungsgegenformation 56 über eine nicht unerhebliche axiale Länge längs der Verteilbahn V gewährleistet, dass der Stopfen 14 nur in einer definierten Relativorientierung in das Verteilkanalbauteil 12 einführbar ist, so dass die Strömungsleitfläche 44 korrekt bezüglich der Abzweigöffnungen 26b und 28b angeordnet ist.

Wegen der spiegelsymmetrischen Ausbildung weist das Verteilkanalbauteil 12 auch im Längsendbereich der Kopplungsöffnung 24 eine Anordnungsgegenformation 60 in Gestalt eines Radialvorsprungs auf, welcher in eine Anordnungsformation 62 des Anschlussstutzens in Gestalt einer Ausnehmung eingreift. Wiederum sind sowohl die Ausnehmung 62 als auch der Radialvorsprung 60 derart ausgebildet, dass sie sich in radialer Richtung zur Verteilkanalbahn V hin verjüngen und sich in axialer Richtung ausgehend von der Kopplungsöffnung 24 zur Längsmitte des Verteilkanalbauteils 12 hin verjüngen. Dies sorgt für eine korrekte Anordnung des Anschlussstutzens 16 im Verteilkanalbauteil 11 und für eine Verdrehsicherung desselben relativ zum Verteilkanal 20 um die Verteilbahn V. Insofern haben die Orientierungsgegenformation 56 und die Anordnungsgegenformation 60 die gegengleiche Gestalt und die gleiche Funktion bzw. Wirkung. Gleiches gilt für die Orientierungsformation 58 einerseits und die Anordnungsformation 62 andererseits.

Das Verteilkanalbauteil 12 ist aus einem weichelastischeren Material hergestellt als der Stopfen 14 und der Anschlussstutzen 16. Das Material des Verteilkanalbauteils 12 weist folglich einen niedrigeren Elastizitätsmodul auf als die Materialien des Stopfens 14 und des Anschlussstutzens 16. Auch dies erleichtert ein Einführen von Stopfen 14 und Anschlussstutzen 16 in das Verteilkanalbauteil 12 und die Herstellung des Formschluss-Rasteingriffs zwischen den Rastvorsprüngen 40 bzw. 50 und den zugeordneten Rastnuten 38 bzw. 48, ebenso wie die Herstellung des Formschlusseingriffs zwischen den Formationen 58 und 62 einerseits sowie den Gegenformationen 56 und 60 andererseits.

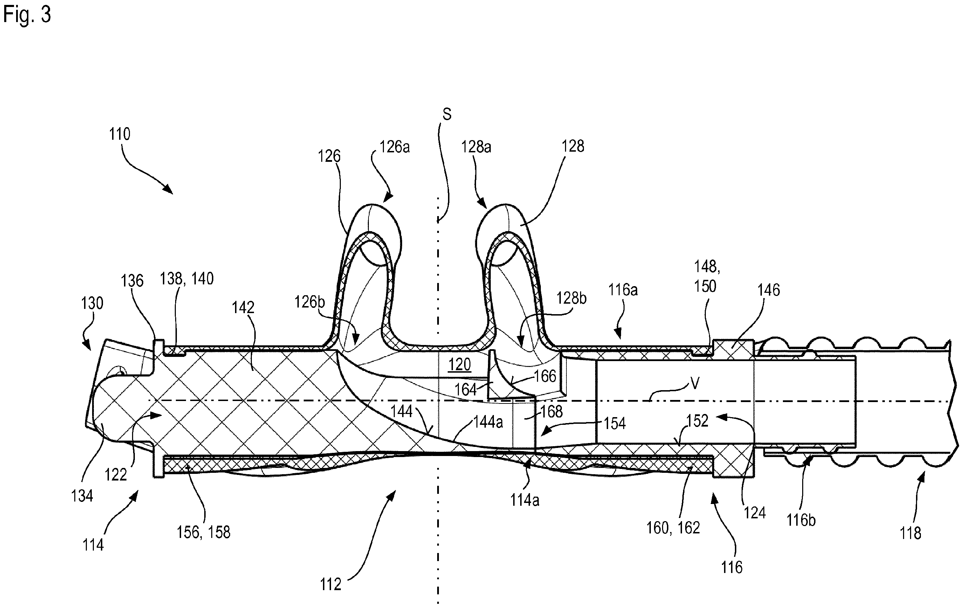

In Fig. 3 ist eine zweite Ausführungsform einer Nasenbrille 110 im selben Längsschnitt dargestellt wie die Nasenbrille 10 von Fig. 1.

Gleiche und funktionsgleiche Bauteile wie in der ersten Ausführungsform der Fig. 1 und 2 sind in der zweiten Ausführungsform der Fig. 3 mit gleichen Bezugszeichen versehen, jedoch erhöht um die Zahl 100.

Die zweite Ausführungsform von Fig. 3 wird nachfolgend nur insofern erläutert, als sie sich von der ersten Ausführungsform der Fig. 1 und 2 unterscheidet, auf deren Beschreibung ansonsten auch zur Erläuterung der zweiten Ausführungsform von Fig. 3 verwiesen wird.

Das Verteilkanalbauteil 112, der Anschlussstutzen 116 und der Zufuhrschlauch 118 sind identisch mit den Bauteilen 12, 16 bzw. 18 der ersten Ausführungsform. Lediglich der Stopfen 114 der zweiten Ausführungsform der Nasenbrille 110 unterscheidet sich vom Stopfen 14 der ersten Ausführungsform.

Zum einen ist in der zweiten Ausführungsform die Strömungsleitfläche 144 längs der Verteilbahn V nur konkav gekrümmt, weist also nur einen einzigen konkaven Flächenabschnitt 144a auf. Dieser erstreckt sich vom Einführlängsende 114a des Stopfens 114 bis zu der dem Stopfen 114 zugeordneten Abzweigöffnung 126b. Jedoch gilt auch für den einzigen um eine zur Verteilbahn V orthogonale Krümmungsachse konkav gekrümmten Abschnitt 144a, dass die Krümmung mit zunehmender Entfernung vom Einführlängsende 114a und mit zunehmender Annäherung an die zugeordnete Abzweigöffnung 126b zunimmt, also ein zugehöriger Krümmungsradius kleiner wird.

Zugleich ist die Strömungsleitfläche 144 auch um eine längs der Verteilbahn V verlaufende weitere Krümmungsachse konkav gekrümmt. Die Strömungsleitfläche 144 bildet daher eine wannenförmige Einfassung eines Strömungsraums im Erstreckungsbereich des Stopfens 114.

Ein weiterer markanter Unterschied des Stopfens 114 zum Stopfen 14 liegt darin, dass an dessen Einführlängsende 114a, gesondert von der ersten Strömungsleitformation 142, eine zweite Strömungsleitformation 164 ausgebildet ist, mit einer zweiten Strömungsleitfläche 166, welche zu der vom Anschlussstutzen 116 bzw. von der Zufuhröffnung 124 her anströmenden Therapiegasströmung freiliegt und durch diese benetzbar ist.

Die zweite Strömungsleitfläche 166 ist im Gegensatz zur zweiachsig gekrümmten ersten Strömungsleitfläche 144 nur einachsig gekrümmt, nämlich nur um eine zur Verteilbahn V orthogonale Krümmungsachse. Dies muss jedoch nicht so sein. Auch die zweite Strömungsleitfläche 166 kann sowohl um eine zur Verteilbahn V orthogonale wie auch um eine zur Verteilbahn V parallele Krümmungsachse gekrümmt sein. Die zweite Strömungsleitfläche 166 befindet sich nur im Längserstreckungsbereich der Abzweigöffnung 128b und ist deshalb dieser zugeordnet.

Die zweite Strömungsleitformation 164 erstreckt sich nur über etwa die halbe Querschnittsfläche des Verteilkanals 120 am Anordnungsort der zweiten Strömungsleitformation 164, so dass zwischen der zweiten Strömungsleitformation 164 und dem Wandungsabschnitt des Verteilkanals 120, welche der der zweiten Strömungsleitfläche 166 zugeordneten Abzweigöffnung 128b diametral gegenüberliegt eine Durchströmöffnung 168 verbleibt. Durch diese Durchströmöffnung 168 strömt von der Zufuhröffnung 124 her anströmendes Therapiegas an der zweiten Strömungsleitformation 164 vorbei und erreicht die erste Strömungsleitfläche 144 und somit die dieser zugeordnete Abzweigöffnung 126b. Die Querschnittsfläche der Durchströmöffnung 168 entspricht etwa der halben Querschnittsfläche des Verteilkanals 120 im Bereich der Anordnung der zweiten Strömungsleitformation 164.

Durch die zweite Strömungsleitformation 164 mit ihrer zweiten Strömungsleitfläche 166 kann ein Teil des im Verteilkanal 120 strömenden Therapiegases gezielt in den einen Versorgungskanal 128 umgelenkt werden, während die erste Strömungsleitfläche 144 den verbleibenden Anteil an Therapiegas in den jeweils anderen Versorgungskanal 126 umlenkt.

Durch die vorgesehenen Strömungsleitflächen 144 und 166 kann Therapiegas weitestgehend verlustfrei, geräuscharm und sehr gezielt zu den einzelnen Abzweigöffnungen 126b und 128b gelenkt werden.

Durch unterschiedlich ausgebildete Stopfen 14 und 114, welche in ein- und dasselbe Verteilkanalbauteil 12 bzw. 112 einführbar sind, können so unterschiedliche Therapiesituationen für den die Nasenbrille 10 bzw. 110 tragenden Patienten realisiert werden, insbesondere Therapiesituationen, bei welchen durch die Abzweigöffnungen 126b und 128b sowie durch die daran anschließenden Versorgungskanäle 126 und 128 gewünscht und gezielt unterschiedlich große Therapiegas-Mengenströme fließen.

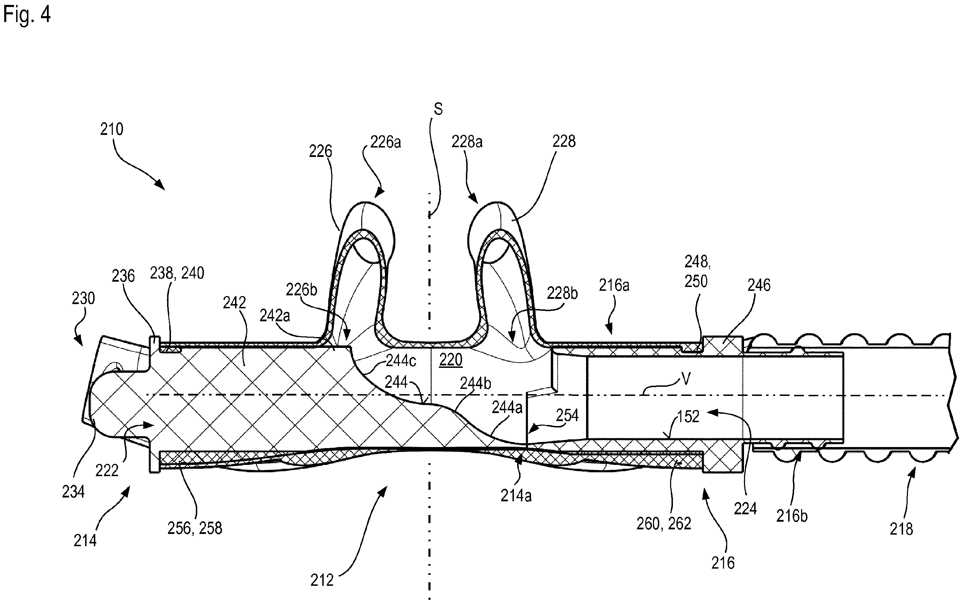

Die Fig. 4 ist eine dritte Ausführungsform in einem der Ansicht der Fig. 2 und 3 entsprechenden Längsschnitt dargestellt.

Gleiche und funktionsgleiche Bauteile und Bauteilabschnitte wie in der ersten Ausführungsform sind in der dritten Ausführungsform mit gleichen Bezugszeichen versehen, jedoch erhöht um die Zahl 200.

Die dritte Ausführungsform der Fig. 4 wird nachfolgend nur insofern erläutert werden, als es sich von der ersten Ausführungsform unterscheidet, auf deren Beschreibung ansonsten auch zur Erläuterung der dritten Ausführungsform von Fig. 4 verwiesen wird.

Wiederum sind das Verteilkanalbauteil 212, der Anschlussstutzen 216 und der Zufuhrschlauch 218 identisch mit den gleichnamigen Bauteilen der ersten Ausführungsform der Fig. 1 und 2.

Der Stopfen 214 entspricht nahezu dem Stopfen 14 der ersten Ausführungsform. Die einzige Strömungsleitfläche 244 ist nur um Krümmungsachsen gekrümmt, welche orthogonal zur Verteilbahn V und orthogonal zur Zeichenebene der Fig. 4 verlaufen. Wie die erste Ausführungsform auch weist die Strömungsleitfläche 244 drei unterschiedlich gekrümmte Krümmungsbereiche auf, nämlich einen dem Einführlängsende 214a nächstgelegenen konkaven Flächenabschnitt 244a, einen sich an diesen längs der Verteilbahn V anschließenden konvexen Flächenabschnitt 244b und einen sich an diesen längs der Verteilbahn V anschließenden weiteren konkaven Flächenabschnitt 244c, welcher bis zur zugeordneten Abzweigöffnung 226b reicht.

Allerdings ist die Strömungsleitfläche 244 des Stopfens 214 der dritten Ausführungsform axial, also längs der Verteilbahn V, kürzer ausgebildet als die Strömungsleitfläche 44 der ersten Ausführungsform. Da der Abstand des Einführlängsendes 214a von der Stoppscheibe 236 des Stopfens 214 der gleiche ist wie bei der ersten Ausführungsform des Stopfens 14, ist durch die Strömungsleitformation 242 ein Sperrabschnitt 242a gebildet, welcher die zugeordnete Abzweigöffnung 226b teilweise verschließt, so dass die Abzweigöffnung 226b einen kleineren wirksamen Strömungsquerschnitt aufweist als die nach wie vor unverschlossene und unverdeckte andere Abzweigöffnung 228b.

Mit der in Fig. 4 gezeigten dritten Ausführungsform kann gewünscht eine verschieden große Menge an Therapiegas pro Zeiteinheit durch die konstruktiv gleich großen Abzweigöffnungen 226b und 228b des Verteilkanalbauteils 212 geleitet werden, so dass einem Patienten gewollt und gezielt über die Versorgungskanäle 226 und 228 verschieden große Therapiegas-Mengenströme verabreicht werden können.

Es ist ohne weiteres vorstellbar, dass auch der Stopfen 214 zusätzlich eine zweite Strömungsleitformation aufweist, wie der Stopfen 114.

Weiterhin kann daran gedacht sein, verschieden große Therapiegas-Massenströme durch die Abzweigöffnungen dadurch zu erzeugen, dass die Durchströmöffnung (siehe Durchströmöffnung 168 in Fig. 3) der zweiten Strömungsleitformation kleiner oder größer als der halbe Strömungsquerschnitt des Verteilkanals ausgebildet ist.

Auch diese Maßnahmen: unterschiedlich große Durchströmöffnung und Sperrabschnitt 242a der Strömungsleitformation 242 können gemeinsam an ein- und demselben Stopfen angewendet werden, um gezielt unterschiedlich große Therapiegas-Mengenströme durch die Abzweigöffnungen 226b und 228b zu erzeugen.

An den Einführlängsenden 14a, 114a und 214a der Stopfen 14, 114 und 214 kann ein Vorsprung oder eine Ausnehmung über einen vorbestimmten Winkelbereich ausgebildet sein, welcher Vorsprung parallel zur Verteilbahn V vom Einführlängsende auskragend in eine Ausnehmung am vorauseilenden Längsende des Einführabschnitts 16a, 116a, 216a des Anschlussstutzens 16, 116, 216 einragt, oder in welche parallel zur Verteilbahn V vom Einführlängsende eingerückte Ausnehmung ein entsprechender Vorsprung am vorauseilenden Längsende des Anschlussstutzens einragt, um den Stopfen und den Anschlussstutzen auch relativ zueinander und nicht nur jeweils relativ zum Verteilkanalbauteil gegen eine Verdrehung um die Verteilbahn V zu sichern.

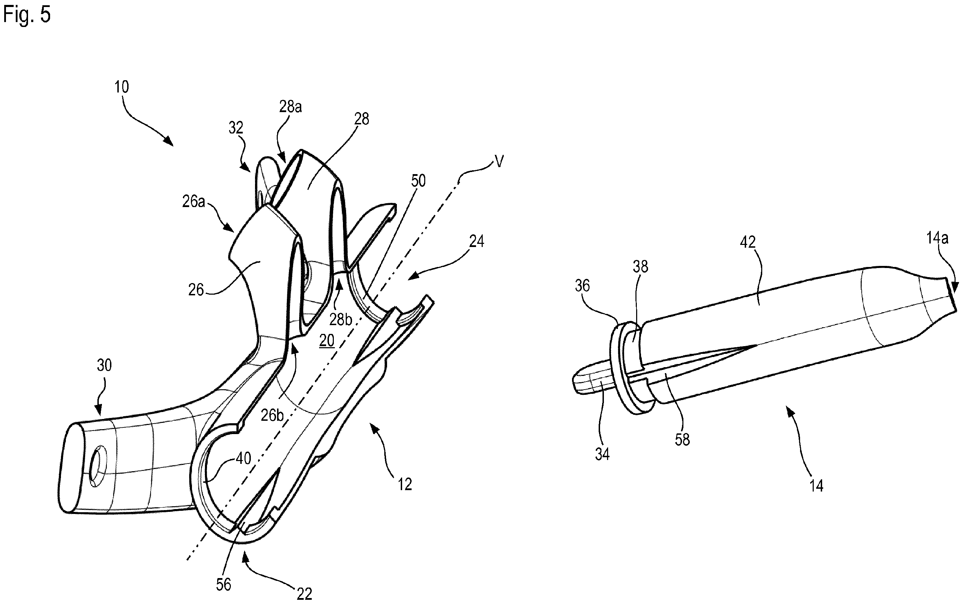

In Fig. 5 ist das teilweise geschnittene Verteilkanalbauteil 12 gezeigt. Hier sind vor allem die Orientierungsgegenformation 56 und die Anordnungsgegenformation 60 im Verteilkanal 20 zu erkennen. Außerdem ist der Stopfen 14 in einer Unteransicht perspektivisch dargestellt, so dass hier die Orientierungsformation 58 in ihrer sich sowohl axial als auch radial verjüngenden Gestalt gezeigt ist. Aus Symmetriegründen weist die Anordnungsformation 62 am Anschlussstutzen die gleiche Gestalt auf.

Eine Nasenbrille 10 kann unterschiedliche Stopfen 14, 114 und 214 aufweisen, so dass durch Wahl eines verwendeten Stopfens aus einer Mehrzahl verfügbarer Stopfen eine konkrete Therapiesituation geschaffen werden kann. So kann ein einfach und schnell konfigurierbarer Nasenbrillen-Therapiebausatz für therapeutisch arbeitendes Personal bereitgestellt werden.

## Patentansprüche

1. Nasenbrille (10; 110; 210) zur nasalen Versorgung eines Patienten mit Therapiegas, umfassend ein Verteilkanalbauteil (12; 112, 212) mit einem Verteilkanal (20; 120; 220), welcher längs einer virtuellen, ihn zentral durchsetzenden Verteilbahn (V) verläuft, wobei die Verteilbahn (V) eine Längsrichtung definiert, wobei der Verteilkanal (20; 120; 220) zwei Abzweigöffnungen (26b, 28b; 126b, 128b; 226b, 228b) aufweist, wobei ausgehend von jeder Abzweigöffnung (26b, 28b; 126b, 128b; 226b, 228b) je ein Versorgungskanal (26, 28; 126, 128; 226, 228) zur Versorgung des Patienten mit Therapiegas vom Verteilkanal (20; 120; 220) abzweigt, wobei der Verteilkanal (20; 120; 220) zusätzlich zu den Abzweigöffnungen (26b, 28b; 126b, 128b; 226b, 228b) zwei mit Abstand von den Abzweigöffnungen (26b, 28b; 126b, 128b; 226b, 228b) angeordnete Kopplungsöffnungen (22, 24; 122, 124; 222, 224) aufweist, wobei die Nasenbrille (10; 110; 210) einen Stopfen (14; 114; 214) umfasst, durch welchen eine der beiden Kopplungsöffnungen (22, 24; 122, 124; 222, 224) als Verschlussöffnung (22; 122; 222) durch den Stopfen (14; 114; 214) für eine Gasdurchströmung verschließbar ist und wobei eine weitere der beiden Kopplungsöffnungen (22, 24; 122, 124; 222, 224) als Zufuhröffnung (24; 124; 224) zum Anschluss einer Gas-Zufuhrleitung (16, 18; 116, 118; 216, 218) an den Verteilkanal (20; 120; 220) ausgebildet ist,
**dadurch gekennzeichnet, dass** der Stopfen (14; 114; 214) eine Strömungsleitformation (42; 142; 242) mit einer freiliegenden Strömungsleitfläche (44; 144; 244) aufweist, wobei die Strömungsleitfläche (44; 144; 244) in einem Bezugszustand, in dem der Stopfen (14; 114; 214) bestimmungsgemäß die Verschlussöffnung (22; 122; 222) verschließend an der Nasenbrille (10; 110; 210) angeordnet ist, im Verteilkanal (20; 120; 220) im Längserstreckungsbereich wenigstens einer Abzweigöffnung (26b, 28b; 126b, 128b; 226b, 228b) angeordnet ist und derart zur Verteilbahn (V) angestellt ist, dass sie im Verteilkanal (20; 120; 220) von der Zufuhröffnung (24; 124; 224) her längs der Verteilbahn (V) anströmendes Therapiegas zu wenigstens einer der Abzweigöffnungen (26b, 28b; 126b, 128b; 226b, 228b) hin umlenkt.

2. Nasenbrille (10; 110; 210) nach Anspruch 1,
**dadurch gekennzeichnet, dass** die Strömungsleitfläche (44; 144; 244) eine konkave Krümmung aufweist.

3. Nasenbrille (10; 110; 210) nach Anspruch 2,
**dadurch gekennzeichnet, dass** die Strömungsleitfläche (44; 144; 244) um eine zur Verteilbahn orthogonale Krümmungsachse (K) wenigstens abschnittsweise konkav oder/und konvex gekrümmt ist.

4. Nasenbrille (110) nach Anspruch 3,
**dadurch gekennzeichnet, dass** die Strömungsleitfläche (144) auch um eine längs der Verteilbahn (V) verlaufende Krümmungsachse konkav gekrümmt ist.

5. Nasenbrille (10; 110; 210) nach einem der vorhergehenden Ansprüche,
**dadurch gekennzeichnet, dass** die Nasenbrille (10; 110; 210) einen Anschlussstutzen (16; 116; 216) aufweist, welcher zum Anschluss einer Gas-Zufuhrleitung (16, 18; 116, 118; 216, 218) in die Zufuhröffnung (24; 124; 224) einführbar ist, wobei eine eine Gasströmung durch den Anschlussstutzen (16; 116; 216) leitende Innenfläche (52; 152; 252) des Anschlussstutzens (16; 116; 216) bei bestimmungsgemäßer, betriebsbereiter Anordnung an der Nasenbrille (10; 110; 210) mit der Strömungsleitfläche (44; 144; 244) eine kontinuierliche strömungsleitende Fläche (54; 154; 254) bildet.

6. Nasenbrille (10; 110; 210) nach Anspruch 5,
**dadurch gekennzeichnet, dass** der Stopfen (14; 114; 214) eine Formschlussformation aufweist und dass der Anschlussstutzen eine Formschlussgegenformation aufweist, wobei die Formschlussformation und die Formschlussgegenformation im betriebsbereiten Zustand, vorzugsweise nur im betriebsbereiten Zustand miteinander in Formschlusseingriff stehen.

7. Nasenbrille (10; 110; 210) nach Anspruch 5 oder 6,
**dadurch gekennzeichnet, dass** der Anschlussstutzen (16; 116; 216) eine Anordnungsformation (62; 162; 262) aufweist und dass das Verteilkanalbauteil (12; 112; 212) im Bereich der Zufuhröffnung (24; 124; 224) eine Anordnungsgegenformation (60; 160; 260) aufweist, wobei die Anordnungsformation (62; 162; 262) und die Anordnungsgegenformation (60; 160; 260) im betriebsbereiten Zustand, vorzugsweise nur im betriebsbereiten Zustand der Nasenbrille (10; 110; 210) miteinander in Formschlusseingriff stehen.

8. Nasenbrille (10; 110; 210) nach einem der vorhergehenden Ansprüche,
**dadurch gekennzeichnet, dass** der Stopfen (14; 114; 214) eine Orientierungsformation (58; 158; 258) aufweist und dass das Verteilkanalbauteil (12; 112; 212) im Bereich der Verschlussöffnung (22; 122; 222) eine Orientierungsgegenformation (56; 156; 256) aufweist, wobei die Orientierungsformation (58; 158; 258) und die Orientierungsgegenformation (56; 156; 256) im betriebsbereiten Zustand, vorzugsweise nur im betriebsbereiten Zustand der Nasenbrille (10; 110; 210) miteinander in Formschlusseingriff stehen.

9. Nasenbrille (10; 110; 210) nach einem der vorhergehenden Ansprüche, **dadurch gekennzeichnet, dass** die beiden Kopplungsöffnungen (22, 24; 122, 124; 222, 224) insoweit gleichartig ausgebildet sind, dass jede der beiden Kopplungsöffnungen (22, 24; 122, 124; 222, 224) sowohl Verschlussöffnung (22; 122; 222) als auch Zufuhröffnung (24; 124; 224) sein kann.

10. Nasenbrille (210) nach einem der vorhergehenden Ansprüche,
**dadurch gekennzeichnet, dass** der Stopfen (214) im Bezugszustand die der Verschlussöffnung (222) näher gelegene Abzweigöffnung (226b) wenigstens teilweise verschließt.

11. Nasenbrille (110) nach einem der vorhergehenden Ansprüche,
**dadurch gekennzeichnet, dass** die Strömungsleitformation (142) eine erste Strömungsleitformation (142) ist, welche von der Zufuhröffnung (124) her längs der Verteilbahn (V) anströmendes Therapiegas zu einer (126b) der Abzweigöffnungen (126b, 128b) hin umlenkt, wobei der Stopfen (114) eine zweite Strömungsleitformation (164) mit einer zweiten Strömungsleitfläche (166) aufweist, welche längs der Verteilbahn (V) mit Abstand von der ersten Strömungsleitformation (142) angeordnet ist und im Bezugszustand von der Zufuhröffnung (124) her längs der Verteilbahn (v) anströmendes Therapiegas zu der jeweils anderen Abzweigöffnung (128b) hin umlenkt.

12. Nasenbrille (110) nach Anspruch 11,
**dadurch gekennzeichnet, dass** die zweite Strömungsleitformation (164) sich nur über einen Teilquerschnitt des Verteilkanals (120) erstreckt und in ihrem Erstreckungsbereich längs der Verteilbahn (V) anströmendes Therapiegas in die jeweils andere Abzweigöffnung (128b) umlenkt und den außerhalb ihres Erstreckungsbereichs mit dem Verteilkanal (120) eine Durchströmöffnung (168) für eine Strömung von Therapiegas an der zweiten Strömungsleitformation (164) vorbei zur ersten Strömungsleitfläche (144) bildet, durch welche hindurch ein Anteil des von der Zufuhröffnung (124) her anströmenden Therapiegases zur ersten Strömungsleitformation (142) strömen kann.

13. Nasenbrille (110) nach Anspruch 11 oder 12,
**dadurch gekennzeichnet, dass** die zweite Strömungsleitformation (164) über einen vorbestimmten, optional aus mehreren mit Abstand voneinander gelegenen Teilwinkelbereichen gebildeten, Winkelbereich um die Verteilbahn (V) hinweg an einer Innenwandung des Verteilkanals (120) anliegt.

14. Nasenbrille (110) nach Anspruch 12, oder nach den Ansprüchen 12 und 13, **dadurch gekennzeichnet, dass** eine von Therapiegas durchströmbare Querschnittsfläche der Durchströmöffnung (168) größer oder kleiner als die Hälfte Querschnittsfläche des Verteilkanals (120) am Ort der Durchströmöffnung (168) ist.

15. Nasenbrille (10; 110; 210) nach einem der vorhergehenden Ansprüche,
**dadurch gekennzeichnet, dass** das Verteilkanalbauteil (12; 112; 212) aus einem Material gebildet ist, welches einen niedrigeren Elastizitätsmodul aufweist als das Material des Stopfens (14; 114; 214) oder/und des Anschlussstutzens (16; 116; 216), sodass das Verteilkanalbauteil (12; 112; 212) beim Einführen eines Einführbauteils aus Stopfen (14; 114; 214) oder/und Anschlussstutzen (16; 116; 216) in das Verteilkanalbauteil (12; 112; 212) durch das jeweilige Einführbauteil verformbar ist.

16. Nasenbrille (10; 110; 210) nach einem der vorhergehenden Ansprüche,
**dadurch gekennzeichnet, dass** sich die beiden Kopplungsöffnungen (22, 24; 122, 124; 222, 224) an entgegengesetzten Längsenden des Verteilkanals (20; 120; 220) befinden, wobei bevorzugt die Verteilbahn (V) zwischen den beiden Kopplungsöffnungen (22, 24; 122, 124; 222, 224) geradlinig verläuft, wobei bevorzugt zusätzlich oder alternativ die beiden Abzweigöffnungen (26b, 28b; 126b, 128b; 226b, 228b) mittig zwischen den Kopplungsöffnungen (22, 24; 122, 124; 222, 224) gelegen sind.

17. Nasenbrillen-Therapiebausatz, umfassend eine Nasenbrille (10; 110; 210) nach einem der vorhergehenden Ansprüche und wenigstens zwei Stopfen (14; 114; 214), welche sich in der Länge der ersten (42; 142; 242) oder/und der zweiten Strömungsleitformation (164) oder/und in der Gestalt der ersten (44; 144; 244) oder/und der zweiten Strömungsleitfläche (166) oder/und in der Größe oder/und Gestalt einer von der zweiten Strömungsleitformation (164) und dem Verteilkanal (120) gebildeten Durchströmöffnung (168) für eine Strömung von Therapiegas an der zweiten Strömungsleitformation (164) vorbei unterscheiden.

## Claims

1. A nasal cannula (10; 110; 210) for nasally supplying a patient with therapeutic gas, encompassing a distribution conduit component (12; 112; 212) having a distribution conduit (20; 120; 220) that extends along a virtual distribution path (V) passing centrally through it; the distribution path (V) defining a longitudinal direction; the distribution conduit (20; 120; 220) comprising two branch openings (26b, 28b; 126b, 128b; 226b, 228b); a respective supply conduit (26, 28; 126, 128; 226, 228) for supplying the patient with therapeutic gas from the distribution conduit (20; 120; 220) branching off from each branch opening (26b, 28b; 126b, 128b; 226b, 228b); the distribution conduit (20; 120; 220) comprising, in addition to the branch openings (26b, 28b; 126b, 128b; 226b, 228b), two coupling openings (22, 24; 122, 124; 222, 224) arranged at a distance from the branch openings (26b, 28b; 126b, 128b; 226b, 228b); the nasal cannula (10; 110; 210) encompassing a plug (14; 114; 214) with which one of the two coupling openings (22, 24; 122, 124; 222, 224), constituting a closure opening (22; 122; 222), is closable by the plug (14; 114; 214) for a flow of gas through it; and a further one of the two coupling openings (22, 24; 122, 124; 222, 224), constituting a delivery opening (24; 124; 224), being embodied for connection of a gas delivery line (16, 18; 116, 118; 216, 218) to the distribution conduit (20; 120; 220),
**characterized in that** the plug (14; 114; 214) comprises a flow-directing configuration (42; 142; 242) having an exposed flow-directing surface (44; 144; 244); the flow-directing surface (44; 144; 244) being arranged, in a reference state in which the plug (14; 114; 214) is arranged on the nasal cannula (10; 110; 210) so as to close off the closure opening (22; 122; 222) as intended, in the distribution conduit (20; 120; 220) in the longitudinal region of extent of at least one branch opening (26b, 28b; 126b, 128b; 226b, 228b), and being canted with respect to the distribution path (V) in such a way that it deflects therapeutic gas, flowing in the distribution conduit (20; 120; 220) in from the delivery opening (24; 124; 224) along the distribution path (V), to at least one of the branch openings (26b, 28b; 126b, 128b; 226b, 228b).

2. The nasal cannula (10; 110; 210) according to Claim 1,
**characterized in that** the flow-directing surface (44; 144; 244) has a concave curvature.

3. The nasal cannula (10; 110; 210) according to Claim 2,
**characterized in that** the flow-directing surface (44; 144; 244) is concavely and/or convexly curved at least locally around a curvature axis (K) orthogonal to the distribution path.

4. The nasal cannula (10) according to Claim 3,
**characterized in that** the flow-directing surface (144) is also concavely curved around a curvature axis extending along the distribution path (V).

5. The nasal cannula (10; 110; 210) according to one of the preceding claims, **characterized in that** the nasal cannula (10; 110; 210) comprises a connector fitting (16; 116; 216) that is introducible into the delivery opening (24; 124; 224) for connection of a gas delivery line (16, 18; 116, 118; 216, 218), an inner surface (52; 152; 252) of the connector fitting (16; 116; 216) which directs a gas flow through the connector fitting (16; 116; 216), when arranged as intended in operationally ready fashion on the nasal cannula (10; 110; 210), forming a continuous flow-conveying surface (54; 154; 254) with the flow-directing surface (44; 144; 244).

6. The nasal cannula (10; 110; 210) according to Claim 5,
**characterized in that** the plug (14; 114; 214) comprises a positive-engagement configuration; and the connector fitting comprises a counterpart positive-engagement configuration, the positive-engagement configuration and the counterpart positive-engagement configuration being in positive engagement with one another in the operationally ready state, preferably only in the operationally ready state.

7. The nasal cannula (10; 110; 210) according to Claim 5 or 6, **characterized in that** the connector fitting (16; 116; 216) comprises an arrangement configuration (62; 162; 262); and the distribution conduit component (12; 112; 212) comprises a counterpart arrangement configuration (60; 160; 260) in the region of the delivery opening (24; 124; 224), the arrangement configuration (62; 162; 262) and the counterpart arrangement configuration (60; 160; 260) being in positive engagement with one another in the operationally ready state, preferably only in the operationally ready state, of the nasal cannula (10; 110; 210).

8. The nasal cannula (10; 110; 210) according to one of the preceding claims, **characterized in that** the plug (14; 114; 214) comprises an orientation configuration (58; 158; 258); and the distribution conduit component (12; 112; 212) comprises a counterpart orientation configuration (56; 156; 256) in the region of the closure opening (22; 122; 222); the orientation configuration (58; 158; 258) and the counterpart orientation configuration (56; 156; 256) being in positive engagement with one another in the operationally ready state, preferably only in the operationally ready state, of the nasal cannula (10; 110; 210).

9. The nasal cannula (10; 110; 210) according to one of the preceding claims, **characterized in that** the two coupling openings (22, 24; 122, 124; 222, 224) are embodied similarly to the extent that each of the two coupling openings (22, 24; 122, 124; 222, 224) can be both a closure opening (22; 122; 222) and a delivery opening (24; 124; 224).

10. The nasal cannula (210) according to one of the preceding claims, **characterized in that** the plug (214), in the reference state, at least partly closes off the branch opening (226b) located closer to the closure opening (222).

11. The nasal cannula (110) according to one of the preceding claims, **characterized in that** the flow-directing configuration (142) is a first flow-directing configuration (142) that deflects therapeutic gas, flowing in from the delivery opening (124) along the distribution path (V), to one (126b) of the branch openings (126b, 128b); the plug (114) having a second flow-directing configuration (164) having a second flow-directing surface (166) that is arranged at a distance along the distribution path (V) from the first flow-directing configuration (142) and that, in the reference state, deflects therapeutic gas, flowing in from the delivery opening (124) along the distribution path (V), to the respective other branch opening (128b).

12. The nasal cannula (110) according to Claim 11,
**characterized in that** the second flow-directing configuration (164) extends over only a partial cross-section of the distribution conduit (120) and, in its region of extent, deflects a therapeutic gas flowing in along the distribution path (V) into the respective other branch opening (128b), and outside its region of extent constitutes, with the distribution conduit (120), a flowthrough opening (168) for a flow of therapeutic gas past the second flow-directing configuration (164) to the first flow-directing surface (144), through which a portion of the therapeutic gas flowing in from the delivery opening (124) can flow to the first flow-directing configuration (142).

13. The nasal cannula (110) according to Claim 11 or 12,
**characterized in that** the second flow-directing configuration (164) abuts against an inner wall of the distribution conduit (120) over a predetermined angular region around the distribution path (V) which is optionally constituted from several angular sub-regions located at a distance from one another.

14. The nasal cannula (110) according to Claim 12 or according to Claims 12 and 13,
**characterized in that** a cross-sectional area, flowthrough-capable for therapeutic gas, of the flowthrough opening (168) is larger or smaller than half the cross-sectional area of the distribution conduit (120) at the location of the flowthrough opening (168).

15. The nasal cannula (10; 110; 210) according to one of the preceding claims, **characterized in that** the distribution conduit component (12; 112; 212) is constituted from a material that has a lower modulus of elasticity than the material of the plug (14; 114; 214) and/or of the connector fitting (16; 116; 216), so that the distribution conduit component (12; 112; 212) is deformable by the respective insertion component upon introduction of one insertion component from among the plug (14; 114; 214) and/or connector fitting (16; 116; 216) into the distribution conduit component (12; 112; 212).

16. The nasal cannula (10; 110; 210) according to one of the preceding claims, **characterized in that** the two coupling openings (22, 24; 122, 124; 222, 224) are located at opposite longitudinal ends of the distribution conduit (20; 120; 220); the distribution path (V) preferably extending in a straight line between the two coupling openings (22, 24; 122, 124; 222, 224); additionally or alternatively, the two branch openings (26b, 28b; 126b, 128b; 226b, 228b) preferably being located centeredly between the coupling openings (22, 24; 122, 124; 222, 224).

17. A nasal cannula therapy kit encompassing a nasal cannula (10; 110; 210) according to one of the preceding claims, and at least two plugs (14; 114; 214) that differ in terms of the length of the first (42; 142; 242) and/or of the second flow-directing configuration (164), and/or in terms of the conformation of the first (44; 144; 244) and/or of the second flow-directing surface (166), and/or in terms of the size and/or conformation of a flowthrough opening (168), constituted by the second flow-directing configuration (164) and the distribution conduit (120), for a flow of therapeutic gas past the second flow-directing configuration (164).

## Revendications

1. Lunettes nasales (10; 110; 210) pour l'alimentation nasale d'un patient en gaz thérapeutique, comprenant un élément de canal de distribution (12; 112, 212) avec un canal de distribution (20; 120; 220) qui s'étend le long d'une voie de distribution virtuelle (V) le traversant de manière centrale, dans lequel la voie de distribution (V) définit une direction longitudinale, dans lequel le canal de distribution (20; 120; 220) présente deux ouvertures de dérivation (26b, 28b; 126b, 128b; 226b, 228b), dans lequel un canal d'alimentation respectif (26, 28; 126, 128; 226, 228) part de chaque ouverture de dérivation (26b, 28b; 126b, 128b; 226b, 228b) pour alimenter le patient en gaz thérapeutique à partir du canal de distribution (20; 120; 220), dans lequel le canal de distribution (20; 120; 220) présente en plus des ouvertures de dérivation (26b, 28b; 126b, 128b; 226b, 228b) deux ouvertures de couplage (22, 24; 122, 124; 222, 224) disposées à distance des ouvertures de dérivation (26b, 28b; 126b, 128b; 226b, 228b), dans lequel les lunettes nasales (10; 110; 210) comprennent un bouchon (14; 114; 214) à travers lequel l'une des deux ouvertures de couplage (22, 24; 122, 124; 222, 224) peut, en tant que ouverture de fermeture (22; 122; 222), être fermée par le bouchon (14; 114; 214) pour un passage de gaz et dans lequel une autre des deux ouvertures de couplage (22, 24; 122, 124; 222, 224) est réalisée comme ouverture d'alimentation (24 124; 224) pour le raccordement d'une conduite d'alimentation en gaz (16, 18; 116, 118; 216, 218) au canal de distribution (20; 120; 220),
**caractérisé en ce que** le bouchon (14; 114; 214) présente une formation de guidage d'écoulement (42; 142; 242) avec une surface de guidage d'écoulement (44; 144; 244) dégagée, dans lequel la surface de guidage d'écoulement (44; 144; 244) dans un état de référence, dans lequel le bouchon (14; 114; 214) est disposé sur les lunettes nasales (10; 110; 210) en fermant l'ouverture de fermeture (22; 122; 222) conformément à sa destination, est disposé dans le canal de distribution (20; 120; 220) dans la zone d'extension longitudinale d'au moins une ouverture de dérivation (26b, 28b; 126b, 128b; 226b, 228b) et est placée par rapport à la voie de distribution (V) de telle sorte qu'elle dévie dans le canal de distribution (20; 120; 220) le gaz thérapeutique affluant depuis l'ouverture d'alimentation (24; 124; 224) le long de la voie de distribution (V) vers au moins une des ouvertures de dérivation (26b, 28b; 126b, 128b; 226b, 228b).

2. Lunettes nasales (10; 110; 210) selon la revendication 1,
**caractérisées en ce que** la surface de guidage d'écoulement (44; 144; 244) présente une courbure concave.

3. Lunettes nasales (10; 110; 210) selon la revendication 2,
**caractérisées en ce que** la surface de guidage d'écoulement (44; 144; 244) est courbée de manière concave ou/et convexe au moins par sections autour d'un axe de courbure (K) orthogonal à la trajectoire de distribution.

4. Lunettes nasales (110) selon la revendication 3,
**caractérisées en ce que** la surface de guidage d'écoulement (144) est également courbée de manière concave autour d'un axe de courbure s'étendant le long de la voie de distribution (V).

5. Lunettes nasales (10; 110; 210) selon l'une des revendications précédentes, **caractérisées en ce que** les lunettes nasales (10; 110; 210) comprennent une pièce de raccordement (16; 116; 216) qui, pour le raccordement d'une conduite d'alimentation en gaz (16, 18; 116, 118; 216, 218), peut être introduite dans l'ouverture d'alimentation (24; 124; 224), dans lequel une surface intérieure (52; 152; 252) de la pièce de raccordement (16; 116; 216), conduisant un écoulement de gaz à travers la pièce de raccordement (16; 116; 216), forme avec la surface de guidage d'écoulement (44; 144; 244) une surface continue guidant l'écoulement (54; 154; 254) lors d'une disposition conforme et opérationnel aux lunettes nasales (10; 110; 210).

6. Lunettes nasales (10; 110; 210) selon la revendication 5,
**caractérisées en ce que** le bouchon (14; 114; 214) présente une formation de fermeture par complémentarité de forme et **en ce que** la pièce de raccordement présente une contre-formation de fermeture par complémentarité de, la formation de fermeture par complémentarité de forme et la contre-formation de fermeture par complémentarité de forme étant en prise de fermeture de forme l'une avec l'autre à l'état opérationnel, de préférence uniquement à l'état opérationnel.

7. Lunettes nasales (10; 110; 210) selon la revendication 5 ou 6,
**caractérisées en ce que** la pièce de raccordement (16; 116; 216) présente une formation d'agencement (62; 162; 262) et **en ce que** l'élément de canal de distribution (12; 112; 212) présente, dans la zone de l'ouverture d'alimentation (24; 124; 224), une contre-formation d'agencement (60; 160; 260), la formation d'agencement (62; 162; 262) et la contre-formation d'agencement (60; 160; 260) étant en prise par complémentarité de forme l'une avec l'autre à l'état opérationnel, de préférence uniquement à l'état opérationnel des lunettes nasales (10; 110; 210).

8. Lunettes nasales (10; 110; 210) selon l'une des revendications précédentes, **caractérisées en ce que** le bouchon (14; 114; 214) présente une formation d'orientation (58; 158; 258) et **en ce que** l'élément de canal de distribution (12; 112; 212) présente, dans la zone de l'ouverture de fermeture (22; 122; 222) une contre-formation d'orientation (56; 156; 256), la formation d'orientation (58; 158; 258) et la contre-formation d'orientation (56; 156; 256) étant en prise par complémentarité de forme l'une avec l'autre à l'état opérationnel, de préférence uniquement à l'état opérationnel des lunettes nasales (10; 110; 210).

9. Lunettes nasales (10; 110; 210) selon l'une des revendications précédentes, **caractérisées en ce que** les deux ouvertures de couplage (22, 24; 122, 124; 222, 224) sont de conception similaire dans la mesure où chacune des deux ouvertures de couplage (22, 24; 122, 124; 222, 224) peut être à la fois une ouverture de fermeture (22; 122; 222) et une ouverture d'alimentation (24; 124; 224).

10. Lunettes nasales (210) selon l'une des revendications précédentes, **caractérisées en ce que** le bouchon (214) obture au moins partiellement, à l'état de référence, l'ouverture de dérivation (226b) plus proche de l'ouverture de fermeture (222).

11. Lunettes nasales (110) selon l'une quelconque des revendications précédentes,
**caractérisées en ce que** la formation de guidage d'écoulement (142) est une première formation de guidage d'écoulement (142) qui dévie le gaz thérapeutique affluant depuis l'ouverture d'alimentation (124) le long de la voie de distribution (V) vers l'une (126b) des ouvertures de dérivation (126b, 128b), dans lequel le bouchon (114) présente une deuxième formation de guidage d'écoulement (164) avec une deuxième surface de guidage d'écoulement (166), qui est disposée le long de la voie de distribution (V) à distance de la première formation de guidage d'écoulement (142) et qui, dans l'état de référence, dévie le gaz de thérapie affluant le long de la voie de distribution (V) depuis l'ouverture d'alimentation (124) vers l'autre ouverture de dérivation respective (128b).

12. Lunettes nasales (110) selon la revendication 11,
**caractérisées en ce que** la deuxième formation de guidage d'écoulement (164) ne s'étend que sur une section partielle du canal de distribution (120) et dévie du gaz thérapeutique affluant dans sa zone d'extension le long de la voie de distribution (V) vers l'autre ouverture de dérivation respective (128b) et forme, en dehors de sa zone d'extension, avec le canal de distribution (120), une ouverture d'écoulement (168) pour un écoulement de gaz thérapeutique au-delà de la deuxième formation de guidage d'écoulement (164) vers la première surface de guidage d'écoulement (144), à travers laquelle une partie du gaz thérapeutique arrivant de l'ouverture d'alimentation (124) peut s'écouler vers la première formation de guidage d'écoulement (142).

13. Lunettes nasales (110) selon la revendication 11 ou 12,
**caractérisées en ce que** la deuxième formation de guidage d'écoulement (164) s'appuie sur une paroi intérieure du canal de distribution (120) sur une zone angulaire prédéterminée autour de la voie de distribution (V), formée en option de plusieurs zones angulaires partielles situées à distance les unes des autres.

14. Lunettes nasales (110) selon la revendication 12 ou selon les revendications 12 et 13,
**caractérisées en ce qu'**une surface de section transversale de l'ouverture d'écoulement (168) pouvant être traversée par le gaz thérapeutique est supérieure ou inférieure à la moitié de la surface de section transversale du canal de distribution (120) à l'endroit de l'ouverture d'écoulement (168).

15. Lunettes nasales (10; 110; 210) selon l'une des revendications précédentes, **caractérisées en ce que** l'élément de canal de distribution (12; 112; 212) est formé d'un matériau qui présente un module d'élasticité plus faible que le matériau du bouchon (14; 114; 214) ou/et de la pièce de raccordement (16; 116; 216), de sorte que l'élément de canal de distribution (12; 112; 212) peut être déformé lors de l'introduction d'un élément d'introduction parmi le bouchon (14; 114; 214) ou/et la pièce de raccordement (16; 116; 216) dans l'élément de canal de distribution (12; 112; 212) par l'élément d'introduction respectif.

16. Lunettes nasales (10; 110; 210) selon l'une des revendications précédentes, **caractérisées en ce que** les deux ouvertures de couplage (22, 24 122, 124; 222, 224) se trouvent à des extrémités longitudinales opposées du canal de distribution (20; 120; 220), dans lequel de préférence la voie de distribution (V) est située entre les deux ouvertures de couplage (22, 24; 122, 124; 222, 224) s'étendant en ligne droite, dans lequel les deux ouvertures de dérivation (26b, 28b; 126b, 128b; 226b, 228b) sont de préférence situées en plus ou alternativement au milieu entre les ouvertures de couplage (22, 24; 122, 124; 222, 224).

17. Kit de lunettes nasales thérapeutiques comprenant des lunettes nasales (10; 110; 210) selon l'une des revendications précédentes et au moins deux bouchons (14 114; 214) qui s'étendent sur la longueur de la première (42; 142; 242) ou/et de la deuxième formation de guidage d'écoulement (164) ou/et sur la forme de la première (44; 144; 244) ou/et de la deuxième surface de guidage d'écoulement (166) ou/et dans la taille ou/et la forme d'une ouverture d'écoulement (168) formée par la deuxième formation de guidage d'écoulement (164) et le canal de distribution (120) pour un écoulement de gaz thérapeutique passant devant la deuxième formation de guidage d'écoulement (164).
